# FASCICULE DE BREVET EUROPEEN

(11) **EP 0 490 951 B1**
(45) Date de publication et mention de la délivrance du brevet: **24.04.1996**
(21) Numéro de dépôt: 90913490.0
(22) Date de dépôt: 06.09.1990
(51) Int. Cl.: C12N 15/31, C07H 21/04, C12Q 1/68

(54) **FRAGMENTS D'ACIDES NUCLEIQUES DERIVES D'UN GENOME DE MYCOBACTERIE APPROPRIEE, LEURS APPLICATIONS DANS LE DIAGNOSTIC DES INFECTIONS A MYCOBACTERIES ET PLASMIDES CONTENANT LESDITS FRAGMENTS**
NUKLEINSÄUREFRAGMENTE VON EINEM GENOM EINES GEEIGNETEN MYKOBAKTERIUMS, IHRE VERWENDUNG ZUR DIAGNOSE VON MYKOBAKTERIENINFEKTIONEN SOWIE DIESE FRAGMENTE ENTHALTENDE PLASMIDE
FRAGMENTS OF NUCLEIC ACIDS DERIVED FROM AN APPROPRIATE MYCOBACTERIA GENOME, THEIR APPLICATIONS IN THE DIAGNOSIS OF MYCOBATERIA INFECTIONS AND PLASMIDES CONTAINING SAID FRAGMENTS

(30) Priorité: 06.09.1989 FR 8911665; 02.03.1990 FR 9002676
(43) Date de publication de la demande: 24.06.1992
(73) Titulaire: INSTITUT PASTEUR, F-75724 Paris Cédex 15 (FR)
(72) Inventeur: GUESDON, Jean-Luc, F-75015 Paris (FR); THIERRY, Dominique, F-92100 Boulogne (FR); ULLMANN, Agnès, F-75016 Paris (FR); GICQUEL, Brigitte, F-75014 Paris (FR); BRISSON-NOEL, Anne, F-75013 Paris (FR)
(74) Mandataire: Ores, Irène
(86) Numéro de dépôt international: FR9000591
(87) Numéro de publication internationale: WO9103558

(56) Documents cités:
- EP-A- 0 272 009
- EP-A- 0 288 306
- WO-A-90/10085
- MOLECULAR MICROBIOLOGY, vol. 3, no. 7, juillet 1989; A.J. HANCE et al., pp. 843-849
- NUCLEIC ACIDS RESEARCH, vol. 18, no. 1, 1990, Oxford University Press; D. THIERRY et al., p. 188
- THE LANCET, vol. 2, no. 8671, 04 novembre 1989; A. BRISSON-NOEL et al., pp. 1069-1071
- INTERNATL. JOURNAL OF LEPROSY, vol. 56, no. 4, 1988, US; P.P. REDDI et al., pp. 592-598
- JOURNAL OF CLINICAL MICROBIOLOGY, vol. 26, no. 11, novembre 1988, American Society for Microbiology, K.D. EISENACH et al., pp. 2240-2245
- MOLECULAR & CELLULAR PROBES, vol. 2, no. 2, 1988, Academic Press Ltd.; R.N. PICKEN et al., pp. 111-124
- TUBERCLE, vol. 69, no. 1, Mars 1988, Longman Group UK Ltd.; CHIA C. PAO et al., pp. 27-36

## Description

La présente Invention est relative à des fragments d'acides nucléiques, dérivés d'un génome de mycobactérie appropriée, notamment de *Mycobacterium tuberculosis,* à leurs applications dans le diagnostic des infections à mycobactéries, ainsi qu'à des plasmides contenant lesdits fragments.

Les mycobactéries correspondent au genre *Mycobacterium* qui comprend au moins 54 espèces différentes.

Parmi celles-ci environ 10 sont pathogènes ou opportunistes pour l'homme ou l'animal. Deux d'entre-elles, *M. tuberculosis* et *M. leprae* sont respectivement les agents de la tuberculose et de la lèpre.

Il est connu que ces deux maladies représentent un problème majeur de Santé Publique ; en effet, il existe actuellement entre 15 et 60 millions d'individus atteints de tuberculose dans le Monde et 2 à 3 millions de personnes meurent chaque année à cause de cette infection. Dans les pays développés, *M. tuberculosis* est la cause la plus commune des infections mycobactériennes. En France, il apparaît environ 10⁴ nouveaux cas de tuberculose par an. La vaccination par le BCG (Bacille de Calmette et Guérin, une souche atténuée de *M. bovis)* est loin d'être efficace au sein de toutes les populations. Cette efficacité varie environ de 80 % dans les pays occidentaux comme l'Angleterre, à 0 % en Inde (résultats du dernier essai de vaccination à Chingleput). De plus, l'apparition de souches de *M. tuberculosis* résistantes aux anti-tuberculeux usuels et l'existence d'une corrélation entre tuberculose et SIDA ajoutent à l'urgence de mettre au point une méthode rapide de détection et d'identification des mycobactéries.

Par exemple, une étude épidémiologique réalisée en Floride a montré que 10 % des malades atteints de SIDA sont atteints de tuberculose au moment du diagnostic du SIDA ou 18 mois avant celui-ci. Chez ces malades, la tuberculose apparaît dans 60 % des cas sous une forme disséminée donc non repérable par les critères de diagnostic classiques comme la radiographie pulmonaire ou l'analyse de crachats.

Enfin, le diagnostic de la tuberculose et des autres mycobactérioses apparentées est difficile à réaliser pour différentes raisons : les maladies pulmonaires causées par différentes mycobactéries ne peuvent pas être distinguées cliniquement, radiologiquement ou histologiquement ; les mycobactéries sont souvent présentes en faible quantité et lorsqu'elles sont en quantité détectable par les méthodes classiquement utilisées, la maladie est déjà en évolution et les malades sont contagieux pour leur entourage ; de plus, en raison du temps de génération très long de ces bactéries (24 h pour *M. tuberculosis* comparé à 20 min pour *E*. *coli),* la culture de ces organismes est difficile. Ainsi faut-il 6 à 8 semaines pour identifier les germes et davantage pour obtenir un antibiogramme utilisable pour le traitement adéquat des malades. Il est donc indispensable de pouvoir disposer d'un test de détection n'exigeant pas de culture des germes et directement utilisable avec les échantillons pathologiques, même lorsque les germes y sont présents à de faibles concentrations.

Plusieurs techniques sont actuellement utilisées en clinique, pour identifier une infection mycobactérienne.

Il faut tout d'abord citer la détection directe des microorganismes au microscope ; cette technique est rapide, mais ne permet pas l'identification de l'espèce mycobactérienne observée et manque de sensibilité dans la mesure où un grand nombre de microorganismes doit être présent dans l'échantillon (> 10⁴/ml) pour permettre une détection fiable (BATES J., CHEST, 1979, 76, (suppl.), 757-763).

Les cultures, lorsqu'elles sont positives, ont une spécificité approchant 100 % et permettent l'identification de l'espèce mycobactérienne isolée ; néanmoins, comme précisé ci-dessus, la croissance des mycobactéries *in vitro* ne peut être réalisée qu'en 3 à 6 semaines et lorsque peu de mycobactéries sont présentes au site de l'infection, des cultures répétées sont nécessaires pour s'assurer d'un résultat positif (BATES J., 1979 et BATES J. et al., Am. Rev. Respir. Dis., 1986, 134, 415-417).

Les techniques sérologiques peuvent s'avérer utiles dans certaines conditions, mais leur utilisation est limitée par leur sensibilité et/ou leur spécificité faibles (DANIEL T.M. et al., Am. Rev. Respir. Dis., 1987, 135, 1137-1151).

La présence ou l'absence de mycobactéries peut également être déterminée par hybridation avec de l'ADN ou de l'ARN en utilisant des sondes spécifiques des séquences d'ADN (KIEHN T.E. et al., J. Clin. Microbiol., 1987, 25, 1551-1552 ; ROBERTS M.C. et al., J. Clin. Microbiol., 1987, 25, 1239-1243; DRAKE T.A. et al., J. Clin. Microbiol., 1987, 25, 1442-1445). Cependant, ces méthodes reposent sur le polymorphisme des séquences nucléotidiques des fragments utilisés ou sur le polymorphisme des régions avoisinantes et nécessitent également la culture des microorganismes.

Certaines séquences d'ADN de différentes mycobactéries et notamment certains gènes codant pour des antigènes mycobactériens ont été décrits. On peut citer notamment la Demande Internationale PCT WO 88/00974, qui a pour Inventeur YOUNG R. et dont le contenu est repris dans un article paru dans Nature, 1985, 316, 450 ; ces publications décrivent les gènes codant pour cinq antigènes de *M. leprae* immunodominants et en particulier le gène codant pour l'antigène de 65 kDA a été séquencé. On peut également citer la Demande Internationale PCT W0 88/05823, qui a pour Co-Inventeurs HUSSON R., YOUNG R. et SHINNICK T. et dont le contenu est repris dans l'article paru dans J. Bact., 1987, 169, 1080-1088 et qui décrit les gènes de *M. tuberculosis* codant pour des antigènes protéiques et notamment pour l'antigène de 65 kDa. Cette Demande Internationale précise, en particulier, que les antigènes de *M. tuberculosis* codant pour cinq protéines immunologiquement actives ont été isolés par un criblage systématique d'une banque d'ADN recombinant exprimée dans un bactériophage lambda gt11, avec une collection d'anticorps monoclonaux dirigés contre les antigènes protéiques de cette bactérie. L'un des antigènes de *M. tuberculosis,* une protéine 65 kDa, présente des déterminants communs à *M. tuberculosis* et *M. leprae.*

La Demande Internationale PCT WO 88/06591, qui a notamment pour Co-Inventeur T. SHINNICK, décrit une protéine recombinante de 540 acides aminés (protéine de 65 kDa) ainsi que la séquence d'ADN et les vecteurs d'expression de ladite protéine, et les applications de ladite protéine recombinée. Cette Demande décrit également des peptides correspondant à des séquences de cette protéine et leurs applications.

Les gènes codant pour les protéines d'autres mycobactéries *(M. africanum, M. smegmatis, M. bovis* BCG et *M. avium)* ont également été isolés. On peut citer notamment THOLE et al. (Infect. Immunol., 1987, 55, 1466-1475), qui ont décrit une protéine de 64 kDa de *M. bovis* BCG exprimée dans *E*. *coli.*

Cependant, les quantités d'ADN mycobactérien présentes dans la plupart des échantillons biologiques sont insuffisantes pour donner un signal positif ; la technique d'hybridation s'est donc révélée inadaptée à la détection d'ADN mycobactérien extrait directement d'échantillons biologiques.

Un certain nombre d'études ont également montré une certaine homologie de structure entre les différentes mycobactéries. Cependant, des différences dans la séquence d'ADN de *M. tuberculosis* et *M. bovis* ont été décrites dans la région 3' du cadre ouvert de lecture de l'antigène 65 kDa (SHINNICK et al., 1987, THOLE et al., 1987), mais une région homologue n'a pas été observée dans l'ADN de *M. leprae* (MEHRA et al., Proc. Nat. Acad. Sci. USA, 1986, 83, 7013-7017, également PCT 88/000974).

Il existe également des publications qui ont mis en évidence l'existence de séquences répétées chez les mycobactéries ; on peut citer en particulier l'article au nom de K.D. EISENACH et al (J. Clin. Microbiol., 1988, 26, 11, 2240-2245) qui décrit trois segments clonés d'ADN de *M. tuberculosis,* qui ont été identifiés par hybridation sélective avec de l'ADN de *M. bovis.* Ces trois segments recombinants, dénommés respectivement M13KE37 (790 paires de bases), M13KE49 (570 paires de bases) et M13KE115 (environ 1600 paires de bases) sont obtenus par clonage dans le bactériophage M13 de fragments d'ADN de *M. tuberculosis.* Toutefois, cet Article fait apparaître que la séquence nucléotidique des segments correspondants n'est pas connue de ses Auteurs.

On peut également citer l'article de D.M. COLLINS et al. (FEMS Microbiol. Letters, 1989, 60, 175-178), qui décrit l'identification d'une séquence d'ADN répétitive spécifique de *M. paratuberculosis.*

Il convient d'autre part de citer l'Article de REDDI et Al. (INTERNATIONAL JOURNAL OF LEPROSY, 1988, 56, N° 4, p. 592-598) qui décrit l'existence dans l'ADN de *Mycobacterium tuberculosis* H37Rv et H37Ra, de fragments répétés dont la séquence n'a pas été déterminée. Les fragments répétés décrits par REDDI et Al., qui sont respectivement des fragments de 5, 6 et 4, 8 Kb, ne sont pas spécifiques du groupe des bacilles de la tuberculose puisqu'ils sont aussi présents chez *M. kansasii* qui n'appartient pas au complexe *tuberculosis.*

Les références complémentaires suivantes constituent également l'état de l'Art préalable à la présente Invention.

BAESS I., Acta Path. Microbiol. Scand., 1979, 87, 221-226 ; BEAUCAGE S.L. et al., Tetrahedron Lett., 1981, 22, 1859-1862 ; EISENACH K.D. et al., Am. Rev. Respir. Dis., 1986, 133, 1065-1068 ; GHEORGHIU M. et al., J. Biol. Standardization, 1988, 16, 15-26 ; GLASSROTH J. et al., N. Engl. J. Med., 1980, 302, 1441-1450 ; HAWKINS C.C. et al., Ann. Intem. Med., 1985, 105, 184-188 ; IMAEDA T., Int. J. Systematic Bacteriol., 1985, 35, 147-150 ; IMAEDA T. et al., Int. J. Systematic Bacteriol., 1988, 38, 151-156; KOGAN S.C. et al., N. Engl. J. Med., 1987, 317, 985-990 ; LI H. et al., Nature (Lond.), 1988, 335, 414-417 ; LU M.C. et al., Infect. Immun., 1987, 55, 2378-2382 ; MANIATIS T. et al., 1982, Cold Spring Harbor, New York ; McFADDEN J.J. et al., Mol. Microbiol., 1987, 1, 283-291 ; PAO C.C. et al., Tubercle, 1988, 69, 27-36 ; PATEL R., J. Gen. Microbiol., 1986, 132, 541-541-551 ; SAIKI R.K. et al., Science, 1988, 239, 487-491 ; SANGER F. et al., Proc. Natl. Acad. Sci. USA, 1977, 74, 5463-5467 ; SMIDA J. et al., Int. J. Leprosy, 1988, 56, 449-454 ; THEIN S.L. et al., in Human Genetic Diseases, 1986, IRL Press, 33-50; THOLE J.E.R. et al., Infect. Immun., 1985, 50, 800-806 ; WATSON E.A., Canad. J. Pub. Health, 1935, 26, 268-275 ; WOLINSKY E., Am. Rev. Respir. Dis, 1979, 119, 107-159.

Récemment un procédé de détection de faibles quantités de mycobactéries par amplification et hybridation directement sur des échantillons biologiques a été mis au point ; ledit procédé utilise le polymorphisme des séquences nucléotidiques d'un fragment de gène commun à toutes les mycobactéries, et notamment un fragment du gène codant pour la protéine 65 kD (Demande de Brevet français n° 89 05057).

Poursuivant les travaux sur les mycobactéries dans cette voie, les Inventeurs ont mis au point un procédé de détection spécifique et encore plus rapide car permettant d'identifier dans un échantillon biologique une mycobactérie d'un groupe donné en moins de 24 heures.

La présente Invention s'est, en conséquence, donné pour but de pourvoir à un procédé de détection et/ou d'identification spécifique d'au moins un groupe de mycobactéries, notamment spécifique du groupe du bacille de la tuberculose permettant de détecter de faibles quantités d'ADN extrait des germes eux-mêmes en nombre restreint et de révéler la présence des mycobactéries du/des groupes à détecter directement dans les échantillons pathologiques.

C'est également un but de l'Invention de pourvoir à des réactifs de diagnostic spécifiques de groupe de mycobactéries et notamment du groupe du bacille de la tuberculose.

La présente Invention a pour objet une séquence nucléotidique issue mycobactéries, caractérisée en ce qu'elle est constituée par une séquence nucléotidique répétée dans le génome d'une mycobactérie et spécifique du groupe du bacille de la tuberculose et en ce qu'elle s'hybride très fortement avec *M. tuberculosis,* c'est-à-dire que, lorsqu'elle est marquée au phosphore 32, elle fournit une tache visible à l'oeil nu après autoradiographie d'une heure et en ce qu'elle s'hybride très faiblement avec M. bovis BCG, c'est-à-dire que, lorsqu'elle est marquée au phosphore 32, elle fournit une tache visible à l'oeil nu après autoradiographie de 48 heures.

Dans la présente Invention, on appelle groupe du bacille de la tuberculose, le groupe qui comprend *M. bovis-*BCG*, M. bovis, M. tuberculosis, M. africa-num, M. microti.*

On entend par séquence nucléotidique, dans la présente Invention, aussi bien une séquence d'ADN double brin, une séquence d'ADN simple brin que les produits de transcription desdites séquences.

On entend par forte hybridation, au sens de la présente Invention, une hybridation donnant un signal important lié au nombre de séquences répétées présentes sur l'ADN génomique ; un signal important permet d'obtenir une tache visible facilement à l'oeil nu après une autoradiographie d'une durée courte, réalisée dans les conditions définies à l'exemple 1 ci-après, notamment inférieure ou égale à une heure ; on obtient, par exemple, une tache très importante après une autoradiographie d'une heure environ, lorsque l'on utilise une sonde marquée ayant une activité spécifique voisine de 10⁹ cpm par µg d'ADN, avec *M. tuberculosis,* alors que pour *M. bovis BCG,* on obtient un signal faible, et ce, seulement après un délai de 48 heures.

Selon un mode de réalisation préféré de ladite séquence, elle compone à son extrémité 5' la séquence 5' TGAACCGCCCCGG 3'de formule I et à son extrémité 3' la séquence 5'CCGGGGCGGTTCA 3' de formule II, ladite séquence contenant en outre au moins un fragment d'une séquence de formule III suivante :

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT

730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TGGGTGTGCC GATCGCCCCA TCGACCTACT ACGACCACAT
850 860 870 880 890 900
[?]AACCGGGAG CCCAGCCGCC GCGAGCTGCG CGATGGCGAA CTCAAGGAGC ACATCAGCCG
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

FORMULE III

Selon une modalité avantageuse de cette disposition, ladite séquence nucléotidique comprend les nucléotides 343-1152 de la séquence de formule III ci-dessus et les fragments de celle-ci, ainsi que les séquences qui présentent au moins 80 % d'homologie avec ladite séquence ou ses fragments.

Selon une autre modalité avantageuse de cette disposition, ladite séquence nucléotidique comprend les nucléotides 327-1684 de la séquence de formule III ci-dessus et les fragments de celle-ci, ainsi que les séquences qui présentent au moins 80 % d'homologie avec ladite séquence nucléotidique ou ses fragments. Cette dernière séquence a été dénommée IS6110 par les Inventeurs et comprend notamment les sites de restriction suivants : Sa II, SmaI, KpnI, HindIII.

La présente Invention englobe également des fragments nucléotidiques, notamment des oligonucléotides, dérivés des séquences nucléotidiques telles que définies ci-dessus, et notamment les oligonucléotides suivants, qui sont caractérisés en ce qu'ils présentent les séquences de formules IV à XVI ci-après, qui sont en outre identifiées par les désignations qui leur ont été attribuées par les Inventeurs : 
ISTB-1 : 5' ATGTCAGGTGGTTCATCGAG 3' (IV)
ISTB-2 : 5' ACAGGCCGAGTTTGGTCATC 3' (V)
ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)
IS-2 : 5' CATCAGCCGCGTCCACGCCGCCAACTACGG 3' (XIV)
IS-4 : 5' TGTGCCGATCGCCCCATCGACCTACTACGA 3' (XV)
IS-6 : 5' GTCGAGTCGATCTGCACACAGCTGACCGAG 3' (XVI)
 ainsi que les séquences qui présentent au moins 80 % d'homologie avec l'un ou l'autre de ces fragments.

L'Invention vise aussi des fragments nucléotidiques complémentaires des précédents, ainsi que des fragments modifiés par rapport aux précédents, par enlèvement ou addition de nucléotide(s) dans une proportion d'environ 15 % par rapport à la longueur des fragments ci-dessus et/ou modifiés au niveau de la nature des nucléotides, dès lors que les fragments nucléotidiques modifiés conservent une capacité d'hybridation avec la séquence d'ADN de mycobactéries, analogue à celle que présentent les fragments correspondants non modifiés.

La présente Invention a également pour objet des produits de traduction et/ou des fragments de ceux-ci, caractérisés en ce qu'ils sont codés par une séquence ou un fragment de séquence nucléotidique conforme à l'Invention.

La présente Invention a, de plus, pour objet un procédé de détection d'une séquence répétée spécifique du groupe du bacille de la tuberculose, caractérisé en ce qu'il comprend :
(1) une étape dans laquelle on réalise une banque de cosmides recombinants comprenant des fragments d'ADN d'une mycobactérie appropriée supérieurs à 30 kb ; et
(2) une étape dans laquelle on détecte le/les clones cosmidiques contenant au moins une séquence répétée, par hybridation avec un ADN génomique total de mycobactérie appropriée, marqué de manière convenable.

La révélation des hybrides formés permet la détection rapide des séquences répétées présentes sur le génome desdites mycobactéries.

La présente Invention a également pour objet des réactifs de diagnostic pour la détection d'au moins un groupe de mycobactéries, caractérisés en ce qu'ils comprennent au moins une séquence nucléotidique ou un fragment de celle-ci, telle que définie ci-dessus, pour la détection des mycobactéries du groupe du bacille de la tuberculose, éventuellement associée à au moins une autre séquence nucléotidique appropriée à la détection d'un autre groupe de mycobactéries et/ou au moins un marqueur approprié.

Un tel réactif permet, par exemple, la détection simultanée d'une mycobactérie du groupe du bacille de la tuberculose et d'une mycobactérie du groupe MAIP (*M. avium, M. intracellulare, M. paratuberculosis).*

Selon un mode de réalisation avantageux desdits réactifs, ils sont constitués par des paires d'amorces pour la synthèse d'un fragment d'ADN ou d'ARN du groupe du bacille de la tuberculose, chaque amorce comprenant une séquence ou un fragment de séquence nucléotidique telle que définie ci-dessus.

Selon une disposition avantageuse de ce mode de réalisation, une paire d'amorces conforme à l'Invention est notamment constituée par un oligonucléotide de formule IV apparié à un oligonucléotide de formule V.

Ces amorces permettent notamment la synthèse de la séquence nucléotidique de formule III ci-dessus et/ou de son brin complémentaire et/ou de la séquence IS6110.

Selon un autre mode de réalisation avantageux desdits réactifs, ils sont constitués par une sonde de détection d'un fragment d'ADN ou d'ARN du groupe du bacille de la tuberculose.

Selon une disposition avantageuse de ce mode de réalisation, ladite sonde de détection comprend avantageusement une séquence nucléotidique de formule III, pour la détection d'une mycobactérie du groupe du bacille de la tuberculose.

Selon un autre mode de réalisation avantageux, le marqueur est choisi dans le groupe qui comprend notamment les isotopes radioactifs, les enzymes appropriées, les fluorochromes, les marqueurs chimiques appropriés, les haptènes et les anticorps ou les analogues de base tels que ceux décrits dans le brevet français n° 2 518 755 ou la demande de brevet européen n° 158 758.

Lesdits réactifs peuvent être utilisés dans un très grand nombre de techniques de diagnostic basées sur la détection d'acides nucléiques par hybridation ; en particulier, une sonde conforme à l'Invention telle que la séquence de formule III permet notamment de détecter spécifiquement l'ADN d'une mycobactérie du groupe du bacille de la tuberculose et en particulier *Mycobacterium tuberculosis.*

La présente Invention a également pour objet une famille de plasmides recombinants, caractérisés en ce qu'ils contiennent au moins une séquence nucléotidique conforme à l'Invention.

Selon un mode de réalisation avantageux dudit plasmide, il comprend la séquence nucléotidique de formule III ou un fragment de celle-ci.

Selon une disposition préférée dudit mode de réalisation, ledit plasmide comprend ladite séquence associée à un vecteur pUC18.

Ce plasmide recombinant a été dénommé pMT01 par les Inventeurs.

Conformément à l'Invention, ledit plasmide recombinant a été déposé sous le n° I-900, en date du 25 août 1989, auprès de la Collection Nationale de Cultures de Microorganismes tenue par l'Institut Pasteur.

La présente Invention a également pour objet un procédé de détection et d'identification rapide d'au moins un groupe et/ou une espèce de mycobactéries dans un échantillon biologique, caractérisé en ce qu'il comprend :
(1) une étape dans laquelle l'on met en contact l'échantillon biologique avec au moins un réactif de diagnostic conforme à l'Invention et
(2) une étape dans laquelle on détecte par tout moyen approprié le ou les produits résultant de l'interaction séquence nucléotidique de mycobactérie éventuellement présente-réactif de diagnostic.

Selon un mode de mise en oeuvre avantageux de ce procédé, le/les réactifs de diagnostic de l'étape (1) sont une/des paires d'amorces conformes à l'Invention et permettent l'obtention de produits d'amplification de la séquence nucléotidique à détecter.

L'étape d'amplification est notamment l'une des techniques d'amplification génétique telles que la méthode dite Qβ réplicase (LIZARDI P.M. et al., Biotechnol., 1988, 6) ou la méthode dite P.C.R. (polymerase chain reaction) décrite dans les demandes de brevet européens n° 200 363, n° 201 184 et n° 229 701 déposées par CETUS CO..

Selon une disposition avantageuse de ce mode de mise en oeuvre, les produits d'amplification obtenus en (1) sont détectés par séparation électrophorétique.

Par exemple, si l'on observe la présence d'un fragment d'ADN migrant à l'endroit attendu, on peut conclure que l'échantillon analysé contient de l'ADN du groupe de mycobactéries à détecter.

Selon une autre disposition avantageuse de ce mode de mise en oeuvre, les produits d'amplification obtenus en (1) sont détectés par hybridation entre lesdits produits d'amplification et un réactif de diagnostic conforme à l'Invention marqué de manière appropriée.

Un tel procédé a l'avantage de permettre de réaliser un test sensible et spécifique, direct et rapide (moins de 24 heures) de détection d'au moins un groupe de mycobactéries.

Selon un mode de mise en oeuvre particulièrement avantageux de ce procédé de détection de la présence de *Mycobacterium tuberculosis* dans un échantillon biologique, il comprend les étapes suivantes :
i) mise en contact de l'échantillon biologique avec un couple de fragments d'acide nucléique, dits amorces, selon l'Invention, l'ADN contenu dans l'échantillon ayant été, le cas échéant, préalablement rendu accessible à l'hybridation et dans ces conditions permettant une hybridation des amorces à l'ADN de *Mycobacterium tuberculosis ;*
ii) amplification de l'ADN de *Mycobacterium tuberculosis ;*
iii) mise en évidence de l'amplification de fragments d'ADN correspondant au fragment encadré par les amorces, par exemple par électrophorèse sur gel ;
iv) vérification éventuelle de la séquence du fragment amplifié, par exemple par hybridation de sonde spécifique, par séquençage ou par analyse de site de restriction.

Selon un autre mode de mise en oeuvre du procédé de diagnostic *in vitro* d'une infection par des mycobactéries, dans un échantillon biologique déterminé, celui-ci comprend les étapes de :
a) mise en contact de l'acide nucléique des mycobactéries éventuellement présent, de l'échantillon biologique testé, dans des conditions permettant l'accessibilité sous forme d'ADN simple brin, avec au moins un couple d'amorces nucléotidique selon l'Invention, lesdites amorces pouvant hybrider avec l'acide nucléique de mycobactéries s'il est présent, et initier la synthèse du produit d'élongation desdites amorces, chaque brin de fragment d'ADN de mycobactéries servant de matrice lorsqu'il est apparié avec les amorces ;
b) séparation des brins d'ADN synthétisés, de leur matrice ;
c) répétition de la synthèse de produit d'élongation, à partir de chaque brin d'ADN présent à l'issue de l'étape b) et susceptible d'hybrider avec les amorces, jusqu'à l'obtention d'une amplification de l'ADN recherché, suffisante pour être détectée ;
d) mise en contact du produit de l'étape c) avec une sonde nucléotidique dans des conditions permettant de détecter la présence du fragment d'ADN amplifié recherché ;
e) détection des produits de l'hybridation éventuellement formés.

Selon un mode de réalisation préféré du procédé pour le diagnostic *in vitro* ci-dessus défini, la mise en contact de l'échantillon testé est précédée d'une étape de traitement de l'échantillon de façon à en extraire l'acide nucléique.

Selon un autre mode de réalisation préféré, le procédé comporte une étape préalable à la mise en contact avec les amorces, consistant en un traitement de l'acide nucléique de l'échantillon avec une réverse transcriptase, pour obtenir la synthèse d'ADNc à partir de l'ARN éventuellement présent dans l'échantillon testé.

Certains des fragments conformes à l'Invention ont l'avantage tout à fait remarquable de pouvoir être utilisés en tant qu'amorces permettant l'amplification de fragments d'ADN de la séquence de formule III des mycobactéries, notamment du groupe du bacille de la tuberculose.

Ces amorces pour l'amplification de fragments d'ADN de mycobactéries, notamment pour l'amplification de fragments de la séquence précédemment décrite de *Mycobacterium tuberculosis,* sont caractérisées en ce qu'elles correspondent à des fragments nucléotidiques définis ci-dessus, choisis parmi le groupe suivant ou parmi les fragments nucléotidiques complémentaires des suivants, ou encore des fragments modifiés mais néanmoins fonctionnels s'agissant de leur capacité d'hybridation avec ledit fragment d'ADN de mycobactéries en vue de son amplification:

ISTB-1 : 5' ATGTCAGGTGGTTCATCGAG 3' (IV)
ISTB-2 : 5' ACAGGCCGAGTTTGGTCATC 3' (V)
ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)

De façon tout à fait avantageuse, et dans le cadre de leur application à l'amplification de fragments d'ADN, ces amorces sont prises en combinaison deux à deux, de façon à hybrider dans des conditions déterminées avec les extrémités 5' et 3' respectives du fragment choisi d'ADN à amplifier.

Différentes combinaisons de ces amorces permettent d'amplifier des fragments d'ADN de taille inférieure à 350 paires de bases, en particulier inférieure à 300 paires de bases. Ceci est particulièrement intéressant puisque l'amplification est d'autant meilleure que le fragment amplifié est plus court.

Selon un mode de réalisation préféré de l'Invention, les amorces pour l'amplification de fragments d'ADN répondant aux définitions précédentes, sont encore caractérisées en ce qu'elles sont choisies parmi les couples d'amorces suivants : ISTB2 et ISTB4 :

5' ACAGGCCGAGTTTGGTCATG 3' (V) et
5' TCGACCTACTACGACCACAT 3' (VII)
ISTB2 et ISTB5 :
5' ACAGGCCGAGTTTGGTCATG 3' (V) et
5' GTCGAGTCGATCTGCACACA 3' (VIII)
ISTB2 et ISTB7 :
5' ACAGGCCGAGTTTGGTCATG 3' (V) et
ISTB7 : 5' CAGCACGCTAATTAACCCGG 3' (X)

ISTB5 et ISTB6 :
5' GTCGAGTCGATCTGCACACA 3' (VIII) et
ISTB6 : 5' GTTGACGGTTAGCCACACTT 3' (IX)
ISTB6 et ISTB7 :
ISTB6 : 5' GTTCAGGGTTAGCCACACTT 3' (IX) et
ISTB7 : 5' CAGCACGCTAATTAACCCGG 3' (X)

Une paire d'amorces particulièrement avantageuse pour réaliser l'amplification voulue est la paire d'amorces constituée par ISTB2 et ISTB7, compte tenu de sa spécificité pour les mycobactéries du groupe de la tuberculose.

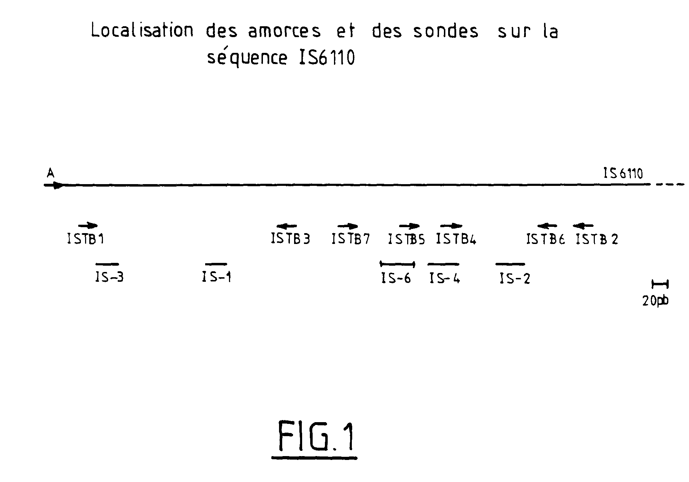

Comme le montre la figure 1 annexée, les couples d'amorces précisés dirigent l'amplification de fragments ayant respectivement les longueurs suivantes :
ISTB1 + ISTB3 dirigent l'amplification d'un fragment de 260 pb,
ISTB2 + ISTB4 dirigent l'amplification d'un fragment de 198 pb,
ISTB2 + ISTB5 dirigent l'amplification d'un fragment de 248 pb,
ISTB5 + ISTB6 dirigent l'amplification d'un fragment de 189 pb,
ISTB6 + ISTB7 dirigent l'amplification d'un fragment de 260 pb,
ISTB2 + ISTB7 dirigent l'amplification d'un fragment de 325 pb.

Les tests réalisés pour évaluer l'efficacité d'amplification obtenue à partir des couples (paires) d'amorces précédemment décrits, montrent que cette efficacité est tout à fait satisfaisante pour les cinq derniers couples d'amorces définis, d'autant plus que les amplifications ont été réalisées sur des dilutions contenant 10ng, 10pg et 10fg d'ADN purifié de *M. tuberculosis,* ce qui montre que l'amplification peut être réalisée à partir de quantités d'ADN extrêmement faibles.

Un autre avantage des amorces proposées dans la présente Invention, réside dans la spécificité des différents couples proposés.

En particulier, certains couples d'amorces permettent de distinguer entre une infection due à des mycobactéries du groupe de la tuberculose ou une infection due à des bactéries atypiques. Ainsi un test mettant en oeuvre différents couples d'amorces a été réalisé sur l'ADN purifié de 19 espèces mycobactériennes comprenant: *M. tuberculosis, M. africanum, M. bovis, M. bovis BCG, M. microti* (ces cinq espèces forment le groupe de la tuberculose), *M. gordonae, M. kansasii, M. malmoense, M. marinum, M. paratuberculosis, M. scrofulaceum, M. simiae, M. szulgai, M. terrae, M. xenopi, M. asiaticum, M. avium, M. chelonae* et *M. flavescens.*

Par exemple, le couple ISTB2/ISTB7, et le couple ISTB1/ISTB2, détectent uniquement les mycobactéries du groupe de la tuberculose, et non les mycobactéries atypiques.

Conformément à l'Invention, les amorces définies dans ce qui précède sont avantageusement marquées par un marqueur chimique, physique ou enzymatique et/ou fixées à un support solide, notamment un support particulaire ou membranaire, par exemple des billes magnétiques.

Selon un autre mode de mise en oeuvre avantageux du procédé conforme à l'Invention, le réactif de diagnostic de l'étape (1) est une sonde de détection conforme à l'Invention et permet l'obtention d'hybrides entre la séquence nucléotidique à détecter et ladite sonde.

Les sondes pour la détection de fragments d'ADN amplifiés de mycobactéries, conformes à l'Invention, sont caractérisées en ce qu'il s'agit de fragments nucléotidiques spécifiques de la séquence de formule III dont on cherche à détecter des fragments, choisis parmi les fragments nucléotidiques appartenant au groupe de ceux qui ont été définis plus haut, capables d'hybrider avec ledit fragment d'ADN amplifié, lesdits fragments étant soit marqués à leur extrémité 5' et/ou 3' par une substance que l'on peut détecter, soit fixés à un support physique.

Comme déjà indiqué plus haut, les marqueurs sont avantageusement choisis parmi les isotopes radioactifs, les enzymes ou marqueurs chimiques appropriés, les fluorochromes, les haptènes, les anticorps, des analogues de bases ou encore un marqueur physique.

La fixation à un support peut être faite sur un support solide particulaire ou membranaire, par exemple des billes magnétiques.

De tels fragments nucléotidiques, doivent être capables d'hybrider avec le fragment d'ADN amplifié, et lorsqu'ils sont marqués par une substance que l'on peut détecter, ils doivent permettre la mise en évidence de la présence d'une infection par les mycobactéries et en particulier celles du groupe de la tuberculose.

Des sondes particulièrement préférées pour la mise en oeuvre de l'Invention sont choisies parmi les fragments nucléotidiques suivants :

IS-2 : 5' CATCAGCCGCGTCCACGCCGCCAACTACGG 3'
IS-4 : 5' TGTGCCGATCGCCCCATCGACCTACTACGA 3'
IS-6 : 5' GTCGAGTCGATCTGCACACAGCTGACCGAG 3'

Les sondes IS-2 et IS-4 ont une taille de 30 bases et elles sont complémentaires des fragments amplifiés à l'aide des paires d'amorces ISTB2/ISTB4, ISTB2/ISTB5, ISTB5/ISTB6, ISTB6/ISTB7 et ISTB2/ISTB7.

La sonde IS-6 est également complémentaire du fragment amplifié à l'aide de la paire d'amorces ISTB2/ISTB4.

Ces sondes permettent avantageusement la détection de tous les membres du groupes des mycobactéries de la tuberculose. Elles présentent également l'avantage pour les quatre dernières paires d'amorces citées, de pouvoir être utilisées simultanément, l'une servant de sonde de capture, et l'autre de sonde de marquage.

Elles permettent donc la réalisation d'une détection directe sur un échantillon biologique en milieu liquide.

Lorsque les sondes sont utilisées comme sondes de marquage, le marqueur peut être choisi parmi le groupe comprenant les isotopes radioactifs, les enzymes appropriées, les fluorochromes ou les marqueurs chimiques ou chimioluminescents appropriés, les haptènes et les anticorps ou les analogues de base tels que ceux décrits dans le Brevet français N° 2 518 755 ou dans la Demande de Brevet européen 158 758, ou encore des marqueurs physiques.

Des marqueurs préférés sont par exemple le phosphore radioactif (³P ) incorporé à l'extrémité 5'.

Lorsque les sondes sont utilisées pour la capture, elles sont avantageusement fixées sur un support solide tel que décrit plus haut.

La séquence IS6110 permet également d'identifier l'espèce d'une mycobactérie à l'intérieur du groupe du bacille de la tuberculose, en partant d'une culture pure.

La présente Invention a, en outre, pour objet un kit, coffret ou ensemble coordonné prêt à l'emploi pour la mise en oeuvre du procédé de détection d'au moins un groupe de mycobactéries conforme à l'Invention, caractérisé en ce qu'il comprend outre des quantités utiles de tampons et de réactifs appropriés pour la mise en oeuvre de ladite détection :
- des doses appropriées d'au moins une paire d'amorces conforme à l'Invention ; et/ou
- des doses appropriées d'au moins une sonde ou un fragment de sonde nucléotidique conforme à l'Invention.

Selon un mode de réalisation avantageux du kit ou nécessaire conforme à la présente Invention, celui-ci comporte les éléments suivants :
- un couple de fragments d'acide nucléique constitué par deux séquences sélectionnées parmi les séquences ISTB définies plus haut;
- les réactifs nécessaires pour effectuer une amplification d'ADN ;
- éventuellement un composant permettant de vérifier la séquence du fragment amplifié, plus particulièrement une sonde nucléique ayant une longueur d'au moins 20 bases capable de s'hybrider avec une partie de la séquence IS6110 se situant entre les deux fragments du couple susdit.

Selon un autre mode de réalisation avantageux du kit pour le diagnostic *in vitro* d'une infection par des mycobactéries, dans un échantillon biologique déterminé, celui-ci comprend :
- au moins une paire d'amorces nucléotidiques correspondant aux définitions données ci-dessus, capable d'hybrider les extrémités 5' et 3' d'un fragment d'ADN spécifique de mycobactéries,
- des réactifs nécessaires à l'extraction des acides nucléiques à partir de l'échantillon traité,
- des réactifs pour effectuer la polymérisation dudit fragment d'ADN, à partir des amorces nucléotidiques, notamment des enzymes de polymérisation, en quantité suffisante pour réaliser l'amplification du fragment d'ADN que l'on souhaite amplifier,
- au moins un fragment nucléotidique pouvant être utilisé comme sonde et capable d'hybrider dans des conditions déterminées avec le fragment d'ADN amplifié,
- un contrôle interne de la réaction d'amplification par exemple constitué par un fragment d'ADN éventuellement porté par un plasmide, ledit fragment pouvant aisément être détecté par hybridation, par exemple en ce qu'il contient un gène de résistance à un antibiotique, ledit fragment étant en outre muni à ses deux extrémités d'au moins une amorce d'amplification, ces amorces étant de préférence choisies parmi les amorces de l'Invention,
- une sonde capable d'hybrider avec le fragment d'ADN contenu dans le contrôle interne,
- le cas échéant, une réverse transcriptase pour obtenir de l'ADNc à partir de l'ARN éventuellement présent dans l'échantillon testé,
- le cas échéant, des moyens pour révéler l'hybridation.

A titre d'échantillon biologique, on peut utiliser tout échantillon de fluide biologique ou de tissu biologique comme par exemple le sang, le sérum, le liquide céphalo-rachidien, le liquide pleural, l'urine, les crachats, les échantillons obtenus par tubage, aspiration bronchique, ponction ou biopsie du foie, biopsie ganglionnaire, etc.

Le kit de diagnostic selon l'Invention a pour avantage de pouvoir être utilisé directement sur des échantillons cliniques et permet d'obtenir des résultats en un temps très rapide.

La présence d'un contrôle interne ajouté à l'échantillon permet de détecter la présence de "faux négatifs" parmi les échantillons. En effet, lorsque la sonde spécifique du contrôle interne ne détecte pas un produit d'amplification, on est vraisemblablement en présence d'un échantillon contenant un inhibiteur de l'ADN-polymérase, inhibiteur qui gêne l'amplification d'ADN ou d'ADNc de mycobactéries. Dans ce cas, différentes dilutions de l'échantillon testé peuvent permettre de mettre en évidence la présence d'acide nucléique de mycobactéries.

Lorsque le témoin inteme présente une réaction positive, une réaction négative au niveau de l'échantillon testé permet de déduire qu'il y a bien absence de mycobactéries.

On note que les amorces incorporées au contrôle interne, ne sont pas nécessairement celles de l'Invention. Cependant, le choix d'autres amorces peut entraîner une diminution de sensibilité.

Selon un mode de réalisation préféré du kit de diagnostic de l'Invention, les amorces utilisées sont ISTB2 et ISTB7 et la sonde de détection du produit éventuel d'amplification est IS-2 éventuellement combinée avec IS-6.

On peut aussi avantageusement utiliser plusieurs sondes de détection et, notamment, ajouter IS-6 à IS-2.

L'Invention vise aussi la production des fragments de nucléotides selon l'Invention, qu'ils soient issus de la séquence IS6110 telle que purifiée à partir des mycobactéries de la tuberculose, ou qu'ils soient synthétisés par voie chimique.

A titre d'exemple, on peut citer pour la synthèse de tels fragments d'acides nucléiques, la méthode au phosphotriester, telle que décrite par NARANG, S.A. et al. dans Meth. of Enzymol., (1979), 68, 90. Une autre méthode adaptée pour la préparation de fragments de nucléotides est la méthode au phosphodiester telle que décrite par BROWN, E.L. et al. dans Meth. Enzymol., (1979), 68, 109.

Cette préparation peut également être effectuée par un processus automatisé, par exemple faisant intervenir les diethylphosphoramidites en tant que constituants de départ, et dans ce cas, la synthèse peut être réalisée suivant la description de BEAUCAGE et al., Tetrahedron Letters, (1981), 22, 1859-1862.

Outre les dispositions qui précèdent, l'Invention comprend encore d'autres dispositions qui ressortiront de la description qui va suivre, qui se réfère à des exemples de mise en oeuvre du procédé objet de la présente Invention.

Il doit être bien entendu, toutefois, que ces exemples sont donnés uniquement à titre d'illustration de l'objet de l'Invention, dont ils ne constituent en aucune manière une limitation.

### EXEMPLE 1 : Dépistage des mycobactéries du groupe du bacille de la tuberculose.

### a) Construction de la banque génomique M. tuberculosis :

L'ADN génomique de *M. tuberculosis* H37rv est digéré partiellement par l'endonucléase de restriction SalI en faisant agir 0,03U d'enzyme par µg d'ADN dans un tampon 100 mM NaCl, 50 mM MgCl₂, 1 mM dithiothréitol pendant 1 heure à 37°C. L'ADN génomique ainsi digéré est séparé par électrophorèse sur gel d'agarose à 0,6 %, les fragments entre 30 et 40 kb électrocutés et précipités en éthanol après extraction au phénol/chloroforme (1/1).

Le vecteur est le cosmide pHC79. Il est digéré de la même manière et déphosphorylé pour éviter toute autoligation.

La ligation s'effectue en mélangeant 700 ng de vecteur et 1,5 µg de fragments d'ADN 30/40 kb (soit un rapport molaire vecteur/insert de 1/2) et la réaction est laissée à 14°C pendant 18 h après avoir ajouté 2,5U de T4 DNA ligase dans un tampon 0,066 M Tris-HCl pH 7,5, 5 mM MgCl₂, 5 mM dithiothréitol, 1 mM ATP.

Les cosmides recombinants sont encapsidés *in vitro* et utilisés pour transformer les bactéries HB101. Les bactéries transformées sont incubées 1 h à 37°C en milieu LB puis étalées sur milieu sélectif Agar contenant 25 µg/ml d'ampicilline. Les colonies résistantes à l'ampicilline sont toutes testées pour leur sensibilité à la tétracycline ; en effet le fragment d'ADN de 30/40 kb est inséré dans le vecteur de manière à inactiver le gène de résistance à la tétracycline (Tet) et à conserver le gène de résistance à l'ampicilline (Amp).

### b) Criblage de la banque et détermination de la séquence :

Il est effectué une mini préparation d'ADN des 150 premières colonies transformantes résistantes à l'ampicilline (Amp^{r}) et sensibles à la tétracycline (tet^{s}) selon la technique de lyse alcaline. L'ADN de ces préparations est ensuite digéré par l'endonucléase de restriction SalI, analysé en électrophorèse sur gel d'agarose à 0,6 % puis transféré sur filtre de nylon. L'ADN est fixé de façon irréversible par 5 mn d'exposition aux UV à 254 nm.

Ces différents filtres sont hybridés 16 à 18 heures à 68°C dans un mélange contenant un tampon 6X SSC (1X SSC correspond à 0,15M NaCl et 0,015M citrate de Na), 10 % dextran sulfate, 5X Denhardt's (une solution 1X Denhardt's correspondant à 0,02 % de Ficoll, 0,02 % de polyvinylpyrrolidone et 0,02 % de sérum albumine bovine), 10 mM EDTA, 0,5 % SDS, 100 µg/ml d'ADN de sperme de saumon ; à ce mélange, on ajoute soit l'ADN génomique total de *M. tuberculosis* H37rv, soit l'ADN génomique total de *M. bovis-BCG* radiomarqué au phosphore 32 par multiamorçage.

Après hybridation, les filtres sont lavés deux fois 10 mn avec du tampon 2X SSC à 65°C, une fois 30 mn avec du tampon 2X SSC+0,1 % SDS à 65°C et enfin une fois 10 mn avec du tampon 0,1X SSC à 65°C. Les filtres encore humides sont mis en autoradiographie à -80°C avec écran intensifiant de 1 h à 2 jours.

Les résultats de ces hybridations ont permis d'isoler un clone cosmidique contenant un fragment d'environ 1 kb s'hybridant très fortement avec l'ADN marqué de *M. tuberculosis* H37rv et faiblement avec l'ADN marqué de *M. bovis-BCG* (figure 1).

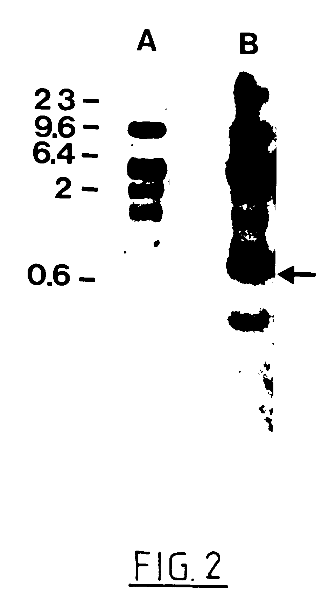

La figure 2 représente une autoradiographie, après Southern Blot, du clone cosmidique, en utilisant les ADN marqués au ³P : la colonne A correspond à *M. bovis-*BCG *;* la colonne B correspond *à M. tuberculosis ; la* flèche montre le fragment spécifique *M. tuberculosis.*

Ce fragment a été cloné dans un vecteur pUC18 et préparé en grande quantité. Ce plasmide est dénommé pMT01.

Ce fragment a été digéré par les enzymes HindIII, KpnI, SmaI et par une double digestion SmaI/HindIII puis cloné dans des phages M13mp18 et M13mp19 et séquencé selon la méthode de Sanger en utilisant la Taq polymérase en présence de d-azaGTP à la place du dGTP.

La séquence entière du fragment est représentée par la formule III ci-dessus.

La comparaison des 1152 premières bases de la séquence ainsi déterminée avec l'ensemble de la banque de données de Los Alamos a fait ressortir des homologies de plus de 50 % avec la séquence d'insertion IS3411 (ISHIGURO et col J. Bacteriol 170, 198, 1902-1906). L'homologie a été détectée entre les nucléotides 330 et 1151.

Deux oligonucléotides de 20 mers à l'intérieur de cette séquence homologue ont été synthétisés :

ISTB1 : ATGTCAGGTGGTTCATCGAG (formule I ci-dessus)
ISTB2 : ACAGGCCGAGTTTGGTCATC (formule II ci-dessus)

### c) Marquage du plasmide pMT01 par 2-acétyl-amino-fluorène (AAF):

25 µg de plasmide pMT01 ont été marqués selon une procédure adaptée de celle de TCHEN et col (PNAS 81 : 1984 ; 3466-3470). Le taux de substitution obtenu a été de 9,7 %.

### d) Préparation d'ADN de mycobactéries

1 ml de culture a été centrifugé 5 mn à 15000 tr/mn, le culot resuspendu dans 200 µl d'eau stérile et soumis à un traitement aux ultra-sons dans un bain sonicant pendant 10 mn. La préparation obtenue a été extraite deux fois par un mélange phénol/chloroforme puis précipitée à l'éthanol. Les culots d'ADN obtenus ont été resuspendus dans 50 µl d'eau stérile.

### e) Amplification de l'ADN :

L'amplification est réalisée par la technique d'amplification *in vitro* selon SAIKI et al (Science, 1988, 239, 487-491) en utilisant 12,5 pmoles des oligonucléotides ISTB1 et ISTB2 et 50 ng d'ADN de différentes souches de mycobactéries, notamment les oligonucléotides TB1 et TB2 tels que décrits dans la Demande de Brevet 89 05057, avec 2 U de Taq polymérase dans un tampon 50 mM KCl, 10 mM Tris-HCl pH 8,3, 1,5 mM MgCl₂, 125 µM de désoxyribonucléotides et 100 µg/ml de gélatine, le volume final de la réaction étant de 100 µl. Les paramètres des étapes de PCR ont été choisis de la façon suivante : 2 mn à 94°C, 2 mn à 60°C, 2mn à 72°C. 40 cycles sont réalisés en utilisant par exemple l'appareil décrit dans le Brevet français n 88 08536 déposé par l'Institut Pasteur. Après le dernier cycle, les échantillons sont maintenus à 72°C 10 mn puis stockés à 4°C.

### f) Analyse des échantillons :

### 1. En gel d'agarose

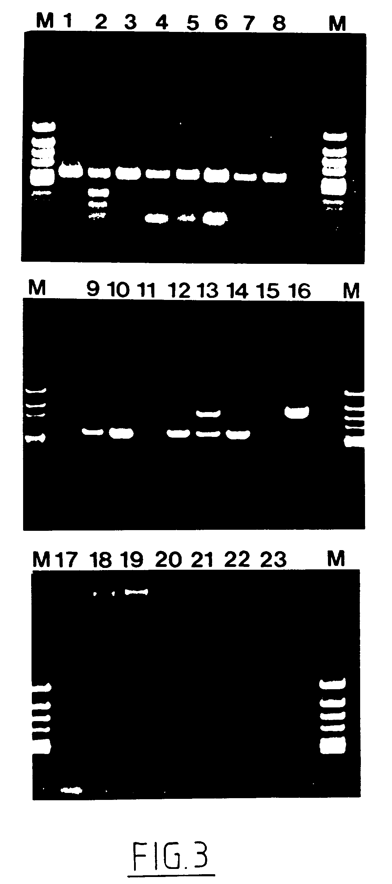

10 µl des échantillons amplifiés sont déposés sur un gel d'agarose à 2 % dans un tampon TBE et contenant 1 µg/ml de bromure d'éthidium. Les bandes amplifiées sont visualisées sous UV. La figure 3 montre les différents profils obtenus selon les ADN de mycobactéries. Un fragment d'ADN correspondant à la taille attendue est observé avec les ADN des mycobactéries suivantes : *M. tuberculosis* (13), *M. bovis-*BCG (16). Par contre ce fragment n'est pas visible lorsque l'ADN analysé est extrait des souches suivantes : *Mycobacterium asiaticum* (1), *M. avium* (2), *M. chelonae* (3), *M. flavescens* (4), *M. gordonae* (5), *M. kansasii* (6), *M. malmoense* (7), *M. marinum* (8), *M. scrofulaceum* (9), M. *simiae* (10), *M.* szulgai (11), *M. terrae* (12), *M. xenopi* (14), *Nocardia* (15), *Streptomyces antibioticus* (17), *S. lividans (18), S. viridochromogene (19), S. hydroscopicus* (20), S. *fradiae* (21), *Micromonospora* (22), *Escherichia coli* (23).

Ce fragment peut éventuellement être détecté par hybridation avec une sonde correspondant à tout ou partie de IS6110. L'ADN est alors transféré sur une membrane et l'on procède comme décrit en b) ci-dessus

### 2. En dot blot

10 µl des échantillons amplifiés sont dénaturés par chauffage à 95°C pendant 2 mn dans 0,2 ml NaOH 0,4 M contenant 25 mM EDTA puis refroidis rapidement sur glace avant d'être déposés dans les puits d'un appareil à filtrer, ajusté avec une membrane de nitrocellulose.

Après lavage des puits avec 100 µl de SSPE, la membrane est chauffée à 80°C pendant 1 heure. Le filtre est hybridé 16 à 18 heures à 68°C avec le plasmide pMT01 marqué à l'AAF. Après lavages la révélation immunoenzymatique s'effectue selon la technique décrite par MASSE et col (Annales de l'Institut Pasteur/Immunology 136D, 231-243). Seules les espèces appartenant au groupe du bacille de la tuberculose donnent un signal sur la membrane.

### EXEMPLE 2 : Identification des différentes espèces à l'intérieur du groupe du bacille de la tuberculose.

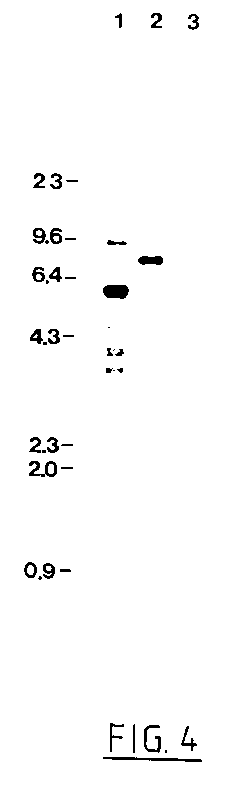

Après culture selon des techniques appropriées, les ADN sont extraits comme décrit au paragraphe 1d. Une digestion totale est effectuée par l'enzyme PstI. Ces ADN subissent ensuite une électrophorèse sur gel d'agarose à 0,6 % en TAE avant d'être transférés sur membrane de nitrocellulose selon la technique de Southern. Une hybridation réalisée dans les conditions décrites ci-dessus et utilisant le plasmide pMT01, ou un plasmide dérivé de celui-ci, marqué par l'acétylamino-fluorène, permet après immunodétection des hybrides formés d'identifier l'espèce de la mycobactérie comme le montre la figure 4 où l'on distingue 14 fragments pour une souche de *M. tuberculosis* (sillon 1) et seulement 2 fragments pour *M. bovis-*BCG (sillon 2), alors que l'on ne distingue aucun fragment au niveau du sillon 3 *(M. avium).*

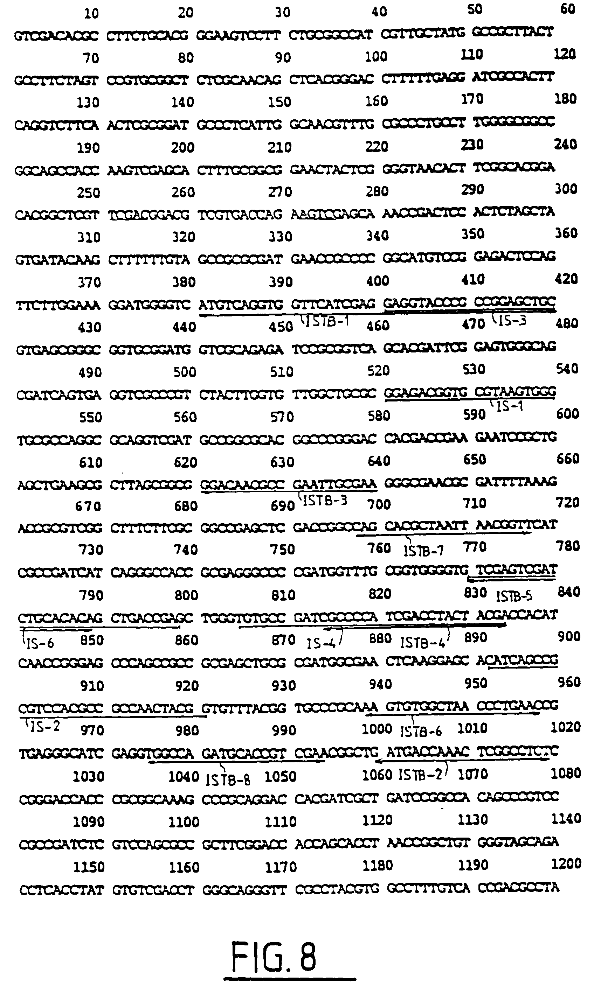

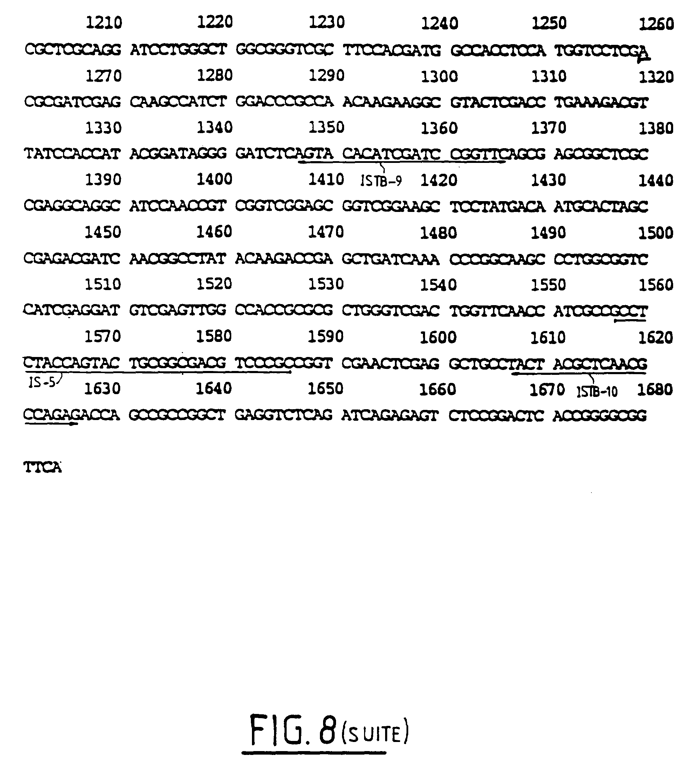

La figure 1 représente la situation des différentes amorces et sondes de formules IV à XVI dans la séquence IS6110, tandis que leurs positions par rapport à la séquence complète de formule III sont représentées à la figure 8.
La figure 5a représente la spécificité d'amplification du couple ISTB2/ISTB7 : l'ARN purifié de 19 espèces mycobactériennes a été amplifié à l'aide de deux couples d'amorces :
   - le couple STB1/STB2 permettant l'amplification d'un fragment (383 pb) du gène codant pour la protéine de 65 kD des mycobactéries,
   - le couple ISTB2/ISTB7,
   les mycobactéries du groupe de la tuberculose présentent une amplification positive avec les deux couples alors que les mycobactéries atypiques ne sont détectées que par le couple TB1/TB2.

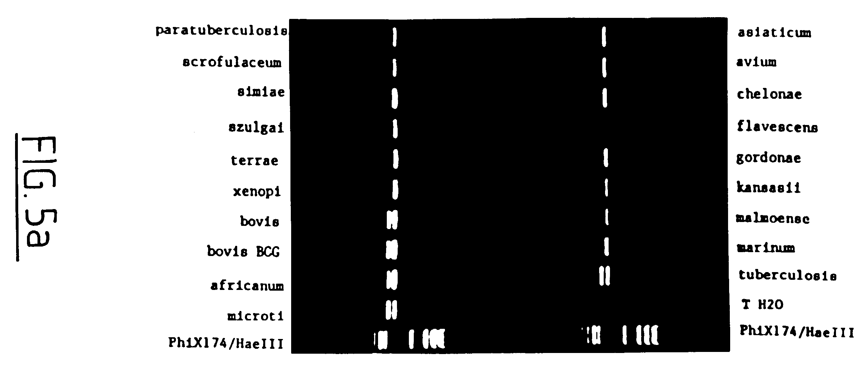

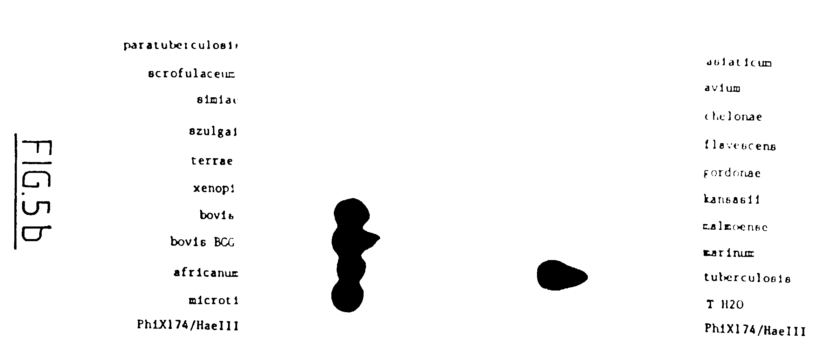

La figure 5b représente l'hybridation de la sonde IS-2 sur le fragment amplifié à partir de ISTB2/ISTB7.

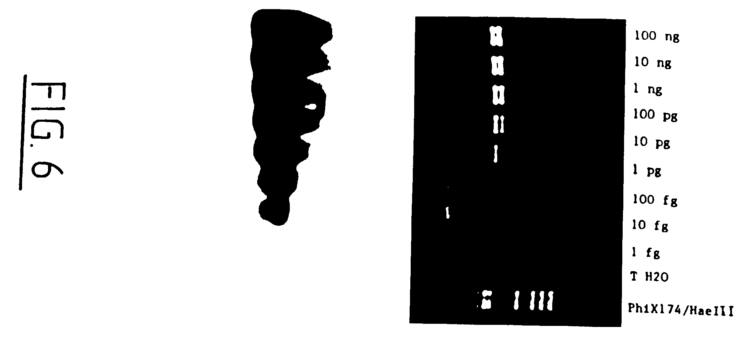

La figure 6 représente la sensibilité de la détection par amplification à l'aide du couple ISTB2/ISTB7 et l'hybridation avec la sonde IS-2.

On peut détecter jusqu'à 10 fg d'ADN de *M. tuberculosis,* ce qui correspond à environ trois bactéries.

### EXEMPLE 3

### Test de détection de M.tuberculosis dans des échantillons cliniques.

Des échantillons cliniques d'origines variées ont été traités suivant les conditions décrites par A.BRISSON-NOEL et al., (THE LANCET, (1989, ii:1069-1071). Une fraction de la préparation d'ADN obtenue a été amplifiée à l'aide des amorces ISTB1/ISTB2 spécifiques de l'antigène 65 kD et des amorces ISTB5/ISTB6 spécifiques de la séquence IS6110. La détection a été réalisée par hybridation avec la sonde IS-4. Les résultats obtenus concordent avec les données cliniques et microbiologiques, ainsi qu'avec les résultats obtenus après hybridation avec une sonde spécifique du gène 65 kD de *M. tuberculosis.*

### Conditions expérimentales

### a) Amplifications

Les réactions d'amplifications sont réalisées d'après la méthode de Saiki et al. (SCIENCE, (1988, 239, 787-491) en utilisant le mélange réactionnel suivant :
- 50 mM KCl,
- 10 mM Tris-HCl pH 8.3,
- 1,5 mM MgCl2,
- 125 µM désoxyribonucléotides,
- 12,5 pmoles oligonucléotides amorces,
- 50 ng ADN,
dans un volume final de 100 µl.

Les cycles de températures utilisées sont les suivants :
- 2 mn à 95°C,
- 2 mn à 60 °C, et
- 2 mn à 72 °C,
pendant 40 cycles, suivis de 5 mn à 72 °C.

### b) Marquage des sondes

Les sondes ont été marquées par incorporation d'un phosphore radioactif (³P) à leur extrémité 5' à l'aide de la polynucléotide kinase: 25 pmoles d'oligonucléotide sonde ont été incubées en présence de Tris-HCl pH 7,5 (50 mM), MgCl2 (10mM) ; dithiotritol (5mM) ; 2,5 ml de (γ³P)-ATP (activité spécifique > 3000 Ci/mmole) et 2 unités de polynucléotide kinase.

### c) Hybridation

Les échantillons amplifiés ont été déposés sur un gel d'agarose et transférés sur une membrane de nylon suivant les techniques classiques (Maniatis). Les hybridations ont été réalisées à l'aide du tampon d'Hybridation rapide Amersham comme suit: préhybridation 15 mn à 65 °C, hybridation 2 heures à 65 °C en présence de 10⁶ cpm de sonde, lavages 2 fois 10 mn en 2 SSC/0,1 % SDS à 20 °C et 1 fois 15 mn en 1 SSC/0,1 % SDS à 65 °C.

### d) Préparation d'ADN de mycobactéries

1 ml de culture a été centrifugé 5 mn à 15000 tr/mn, le culot resuspendu dans 200 µl d'eau stérile et soumis à un traitement aux ultra-sons dans un bain sonicant pendant 10 mn. La préparation obtenue a été extraite deux fois par un mélange phénol/chloroforme puis précipitée à l'éthanol. Les culots d'ADN obtenus ont été resuspendus dans 50 µl d'eau stérile.

### e) Traitement des échantillons

Les échantillons ne sont pas décontaminés avant le traitement. Un aliquot de 200 µl est placé dans un tube Eppendorf, centrifugé et le culot est resuspendu dans 200 µl de solution de lyse (NaOH O,1 N, NaCl 2 M, SDS 0,5 %). Après 15 mn d'incubation à 95 °C, on procède à deux extractions phénol/chloroforme et une précipitation à l'éthanol. Le culot d'ADN est resuspendu dans 50 µl d'eau stérile. Le volume utilisé pour l'amplification est de 5 µl.

Ce procédé de diagnostic peut être appliqué à des quantités d'ADN faibles, et il s'avère donc intéressant pour la réalisation de diagnostics précoces et rapides.

### f) évaluation sur échantillons cliniques (tableau I)

41 échantillons cliniques ont été analysés à l'aide des amorces ISTB2/ISTB7 et de la sonde IS-2. Les mêmes échantillons ont été testés en parallèle par les techniques classiques (examen direct et culture) :
- 16 échantillons positifs en culture ont été trouvés positifs par amplification,
- 18 échantillons négatifs en culture ont été trouvés négatifs par amplification,
- 1 échantillon faiblement positif en culture a été trouvé négatif par amplification ; les Inventeurs ont montré, à l'aide d'un contrôle interne d'amplification (amplification simultanée d'un plasmide ajouté au mélange d'amplification), que cet échantillon contient des inhibiteurs de la réaction d'amplification. Contrairement aux résultats obtenus avec d'autres échantillons qui contenaient également des inhibiteurs, des dilutions successives de cet échantillon sont demeurées négatives,
- 6 échantillons négatifs en culture ont été trouvés positifs par amplification. Ces résultats ne correspondent pas à des faux positifs car les échantillons provenaient de patients ayant effectivement une tuberculose, le diagnostic ayant été fait sur l'existence d'autres prélèvements positifs en culture ou sur des analyses histobiologiques. Ces résultats montrent la bonne sensibilité du test décrit dans la présente demande, par rapport aux techniques classiques. 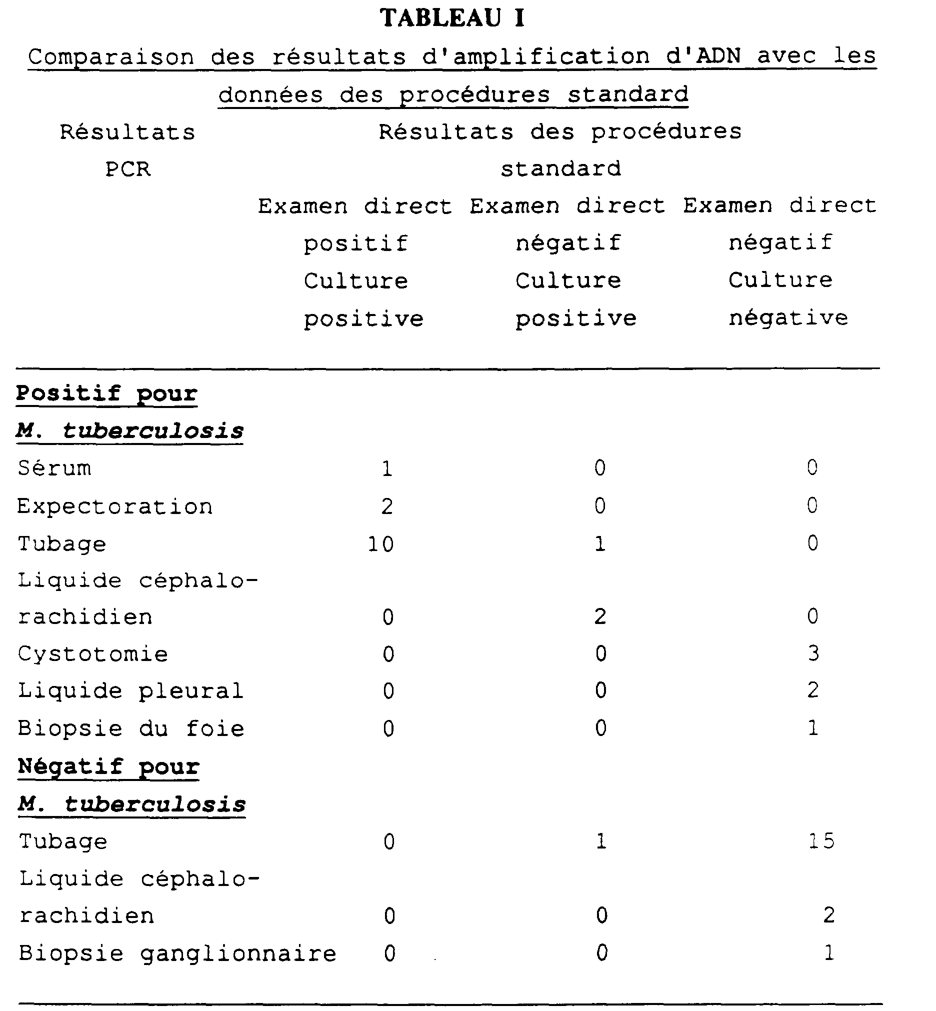 Examen direct : Examen microscopique après coloration de Ziehl-Neelssen ; Culture : culture sur milieu solide de Loewenstein-Jensen.

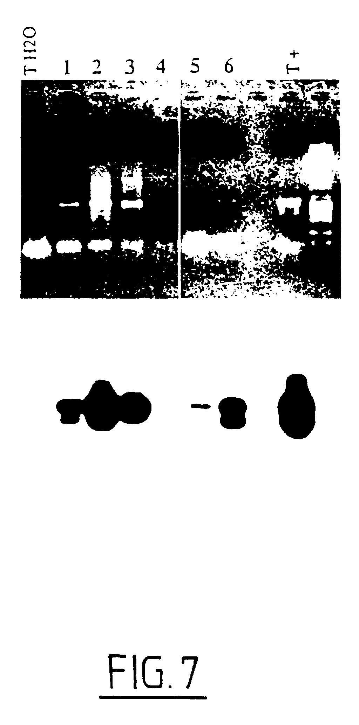

La figure 7 représente la détection de. *M. tuberculosis* dans les échantillons cliniques :
1. tubage (positif en culture),
2. aspiration bronchique (positif à l'examen microscopique et en culture),
3. tubage (positif à l'examen microscopique et en culture),
4. tubage (négatif en culture),
5. liquide céphalorachidien (positif en culture),
6. tubage (positif en culture).

Ainsi que cela ressort de ce qui précède, l'Invention ne se limite nullement à ceux de ses modes de mise en oeuvre, de réalisation et d'application qui viennent d'être décrits de façon plus explicite; elle en embrasse au contraire toutes les variantes qui peuvent venir à l'esprit du technicien en la matière, sans s'écarter du cadre, ni de la portée, de la présente Invention.

## Revendications (Revendications pour l'(les) Etat(s) contractant(s) suivant(s): AT, BE, CH, DE, DK, FR, GB, IT, LI, LU, NL, SE)

1. Séquence nucléotidique issue mycobactéries, caractérisée en ce qu'elle est constituée par une séquence nucléotidique répétée dans le génome d'une mycobactérie et spécifique du groupe du bacille de la tuberculose et en ce qu'elle s'hybride très fortement avec *M. tuberculosis,* c'est-à-dire que, lorsqu'elle est marquée au phosphore 32, elle fournit une tache visible à l'oeil nu après autoradiographie d'une heure et en ce qu'elle s'hybride très faiblement avec M. bovis BCG, c'est-à-dire que, lorsqu'elle est marquée au phosphore 32, elle fournit une tache visible à l'oeil nu après autoradiographie de 48 heures.

2. Séquence selon la Revendication 1, caractérisée en ce qu'elle comporte à son extrémité 5' la séquence 5'TGAACCGCCCCGG 3' de formule I et à son extrémité 3' la séquence 5' CCGGGGCGGTTCA 3' de formule II, ladite séquence contenant en outre au moins un fragment d'une séquence de formule III suivante :

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGGGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTTTA GCCGCCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
CTGCACACAG CTGACCGAGC TGGGTGTGCC GATCGCCGCA TCGACCTACT ACGACCACAT
850 860 870 880 890 900
GAACCGGGAG CCGAGCCGCC GCCAGCTGCG CGATGGCGAA CTCAAGGAGC ACATCAGCCG
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TGGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTCATCAAA CCCGGCAAGC CCTGGCGGTC
1510 1520 1530 1540 1550 1560
CATCGAGGAT GTCGAGTTCG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

Formule III

3. Séquence nucléotidique selon la Revendication 2, caractérisée en ce qu'elle comprend le fragment 343-1152 de la séquence de formule III selon la Revendication 2 et toute séquence présentant au moins 80 % d'homologie avec celle-ci.

4. Séquence nucléotidique selon la Revendication 2, caractérisée en ce qu'elle comprend le fragment 327-1684 de la séquence de formule III selon la Revendication 2 et toute séquence présentant au moins 80 % d'homologie avec celle-ci.

5. Oligonucléotide, caractérisé en ce qu'il est constitué par un fragment d'une séquence nucléotidique selon l'une quelconque des Revendications 1 à 4.

6. Oligonucléotide selon la Revendication 5, caractérisé en ce qu'il présente la séquence de formule IV suivante :

ATGTCAGGTGGTTCATCGAG (IV).

7. Oligonucléotide selon la Revendication 5 caractérisé en ce qu'il présente la séquence de formule V suivante :

ACAGGCCGAGTTTGGTCATC (V).

8. Fragments nucléotidiques selon la Revendication 5, caractérisés en ce qu'ils présentent l'une des séquences suivantes :

ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)
IS-2 : 5' CATCAGCCGCGTCCACGCCGCCAACTACGG 3' (XIV)
IS-4 : 5' TGTGCCGATCGCCCCATCGACCTACTACGA 3' (XV)
IS-6 : 5' GTCGAGTCGATCTGCACACAGCTGACCGAG 3 ' (XVI)

9. Fragments nucléotidiques caractérisés en ce qu'il s'agit de fragments complémentaires des fragments selon la Revendication 8 ou de fragments modifiés par rapport aux précédents, par enlèvement ou addition de nucléotide(s) dans une proportion d'environ 10 à 15 % par rapport à la longueur des fragments ci-dessus et/ou par modification de la nature des nucléotides : dès lors que les fragments nucléotidiques modifiés conservent une capacité d'hybridation avec la séquence d'ADN de mycobactéries, analogue à celle que présentent les fragments correspondants non modifiés.

10. Amorces pour l'amplification de fragments d'ADN de mycobactéries, notamment pour l'amplification de fragments d'une séquence de Mycobacterium tuberculosis, caractérisées en ce qu'elles correspondent à des fragments nucléotidiques selon l'une quelconque des Revendications 6 à 9, choisis parmi les suivants ou parmi leurs fragments nucléotidiques complémentaires :

ISTB1 : 5' ATGTCAGGTGGTTCATCGAG 3'
ISTB2 : 5' ACAGGCCGAGTTTGGTCATC 3'
ISTB3 : 5' TTCGCAATTCTGGCGTTGTCC 3'
ISTB4 : 5' TCGACCTACTACGACCACAT 3'
ISTB5 : 5' GTCGAGTCGATCTGCACACA 3'
ISTB6 : 5' GTTCAGGGTTAGCCACACTT 3'
ISTB7 : 5' CAGCACGCTAATTAACCCGG 3'
ISTB8 : 5' TGGCCAGATGCACCGTCGAA 3'
ISTB9 : 5' AGTACACATCGATCCGGTTC 3'
ISTB10 : 5' TACTACGCTCAACGCCAGAG 3'

11. Amorces pour l'amplification de fragments d'ADN de mycobactéries, selon la Revendication 10, caractérisées en ce qu'elles sont prises en combinaison deux à deux, et en ce qu'il s'agit des couples suivants :

ISTB2 et ISTB4 :
5' ACAGGCCGAGTTTGGTCATC 3' et
5' TCGACCTACTACGACCACAT 3'
ISTB2 et ISTB5 :
5' ACAGGCCGAGTTTGGTCATC 3' et
5' GTCGAGTCGATCTGCACACA 3'
ISTB5 et ISTB6 :
5' GTCGAGTCGATCTGCACACA 3' et
5' GTTCAGGGTTAGCCACACTT 3'
ISTB6 et ISTB7 :
5' GTTCAGGGTTAGCCACACTT 3' et
5' CAGCACGCTAATTAACCCGG 3'.

12. Amorces pour l'amplification de fragments d'ADN selon la Revendication 10 ou la Revendication 11 caractérisées en ce qu'elles correspondent au couple d'amorces suivant :

ISTB2 et ISTB7 :
5' ACAGGCCGAGTTTGGTCATC 3' et
5' CAGCACGCTAATTAACCCGG 3'.

13. Amorces selon l'une quelconque des Revendications 10 à 12, caractérisées en ce qu'elles sont marquées par un marqueur chimique, physique ou enzymatique et/ou en ce qu'elles sont fixées à un support solide, notamment un support particulaire ou membranaire, en particulier des billes magnétiques.

14. Produits de traduction et/ou fragments de ceux-ci, caractérisés en ce qu'ils sont codés par une séquence ou un fragment de séquence nucléotidique selon l'une quelconque des revendications 1 à 4.

15. Sonde nucléique apte à détecter par hybridation la présence d'ADN spécifique du Mycobacterium tuberculosis dans un échantillon biologique, caractérisée en ce qu'elle comporte une séquence ayant une longueur d'au moins 20 bases, capable de s'hybrider, après mise en contact pendant 16 à 18 h à 68°C ou 2 h à 65°C, avec une partie de la séquence constituée par le fragment 327-1684 selon la Revendication 4, se situant entre les deux amorces nucléiques.

16. Sonde selon la Revendication 15, caractérisée en ce qu'il s'agit de l'un des fragments nucléotidiques suivants :

IS-2 : 5' CATCAGCCGCGTCCACGCCGCCAACTACGG 3'
IS-4 : 5' TGTGCCGATCGCCCCATCGACCTACTACGA 3'
IS-6 : 5' GTCGAGTCGATCTGCACACAGCTGACCGAG 3'

17. Sondes pour la détection de fragments d'ADN amplifiés de mycobactéries, selon la Revendication 15, caractérisées en ce qu'il s'agit de fragments nucléotidiques spécifiques d'une séquence de mycobactérie, selon l'une quelconque des Revendications 5 à 8, capables d'hybrider avec ledit fragment d'ADN amplifié après contact pendant 16 à 18 h à 68°C ou 2 h à 65°C, lesdits fragments étant soit marqués à leur extrémité 5' et/ou 3' par une substance que l'on peut détecter, par exemple par un isotope radioactif, une enzyme, un marqueur chimique ou chimioluminescent approprié, un fluorochrome, un haptène, ou un anticorps, des analogues de bases ou encore un marqueur physique, soit fixés à un support solide notamment un support particulaire ou membranaire, par exemple des billes magnétiques.

18. Procédé de détection d'une séquence répétée spécifique du groupe du bacille de la tuberculose, selon l'une quelconque des Revendications 1 à 4, caractérisé en ce qu'il comprend :
(1) une étape dans laquelle on réalise une banque de cosmides recombinants comprenant des fragments d'ADN d'une mycobactérie appropriée supérieurs à 30 kb ; et
(2) une étape dans laquelle on détecte le/les clones cosmidiques contenant au moins une séquence répétée, par hybridation 16 à 18 h à 68°C avec un ADN génomique total de mycobactérie appropriée, marqué de manière convenable.

19. Réactif de diagnostic pour la détection d'au moins un groupe de mycobactéries, caractérisé en ce qu'il comprend au moins une séquence nucléotidique ou un fragment de celle-ci selon l'une quelconque des Revendications 1 à 8 pour la détection des mycobactéries du groupe du bacille de la tuberculose, éventuellement associée à au moins une autre séquence nucléotidique appropriée à la détection d'un autre groupe de mycobactéries et/ou à au moins un marqueur approprié.

20. Réactif selon la Revendication 19, caractérisé en ce qu'il comprend des paires d'amorces pour la synthèse d'un fragment d'ADN ou d'ARN du groupe du bacille de la tuberculose, chaque amorce comprenant une séquence ou un fragment de séquence nucléotidique selon l'une quelconque des Revendications 1 à 8.

21. Réactif selon la Revendication 20, caractérisé en ce qu'il est constitué par une paire d'amorces comprenant des oligonucléotides appariés conformément aux Revendications 10 à 13.

22. Réactif selon la Revendication 19, caractérisé en ce qu'il comprend une sonde de détection appropriée d'un fragment d'ADN ou d'ARN du groupe du bacille de la tuberculose.

23. Réactif selon la Revendication 22, caractérisé en ce que ladite sonde de détection comprend avantageusement une séquence nucléotidique de formule III, selon l'une quelconque des Revendications 2 à 4.

24. Réactif selon la Revendication 19 ou la Revendication 22, caractérisé en ce que le marqueur est choisi dans le groupe qui comprend notamment les isotopes radioactifs, les enzymes appropriées, les fluorochromes, les marqueurs chimiques appropriés, les haptènes et les anticorps ou les analogues de base appropriées.

25. Famille de plasmides recombinants, caractérisés en ce qu'ils contiennent au moins une séquence nucléotidique selon l'une quelconque des Revendications 1 à 4.

26. Plasmide selon la Revendication 25, caractérisé en ce qu'il comprend la séquence nucléotidique de formule III selon la Revendication 2 ou un fragment de celle-ci ou une séquence homologue.

27. Plasmide selon la Revendication 25, caractérisé en ce qu'il comprend ladite séquence associée à un vecteur pUC 18.

28. Plasmide selon la Revendication 26 ou la Revendication 27, caractérisé en ce qu'il a été dénommé pMT01 et en ce qu'il a été déposé sous le n°I-900, en date du 25 août 1989, auprès de la Collection Nationale de Cultures de Microorganismes tenue par l'Institut Pasteur.

29. Procédé de détection et d'identification rapide d'au moins un groupe et/ou une espèce de mycobactéries dans un échantillon biologique, caractérisé en ce qu'il comprend :
(1) une étape dans laquelle l'on met en contact l'échantillon biologique avec au moins un réactif de diagnostic selon l'une quelconque des Revendications 20 à 24,
(2) une étape dans laquelle on détecte par tout moyen approprié le ou les produits résultant de l'interaction séquence nucléotidique de mycobactérie éventuellement présente-réactif de diagnostic.

30. Procédé selon la Revendication 29, caractérisé en ce que le/les réactifs de diagnostic de l'étape (1) sont une/des paires d'amorces selon l'une quelconque des revendications 10 à 13 et permettent l'obtention de produits d'amplification de la séquence nucléotidique à détecter.

31. Procédé selon la Revendication 30, caractérisé en ce que les produits d'amplification obtenus en (1) sont détectés par séparation électrophorétique.

32. Procédé selon la Revendication 30, caractérisé en ce que les produits d'amplification obtenus en (1) sont détectés par hybridation 2 h à 65°C entre lesdits produits d'amplification et un réactif de diagnostic selon l'une quelconque des Revendications 19, 22, 23 et 24.

33. Procédé selon la Revendication 29, caractérisé en ce que le réactif de diagnostic de l'étape (1) est une sonde de détection selon l'une quelconque des Revendications 19, 22, 23 ou 24.

34. Procédé de détection de la présence de Mycobacterium tuberculosis dans un échantillon biologique, selon la Revendication 29, caractérisé par les étapes suivantes :
i) mise en contact de l'échantillon biologique avec un couple de fragments d'acide nucléiques, dits amorces, selon l'une quelconque des Revendications 10 à 13, l'ADN contenu dans l'échantillon ayant été, le cas échéant, préalablement rendu accessible à l'hybridation et dans des conditions permettant une hybridation des amorces à l'ADN de Mycobacterium tuberculosis, telles que 2 min à 60°C ;
ii) amplification de l'ADN de Mycobacterium tuberculosis ;
iii) mise en évidence de l'amplification de fragments d'ADN correspondant au fragment encadré par les amorces, notamment par électrophorèse sur gel ;
iv) vérification éventuelle de la séquence du fragment amplifié, notamment par hybridation de sonde spécifique, par séquençage ou par analyse de site de restriction.

35. Procédé pour le diagnostic in vitro d'une infection par des mycobactéries sur un échantillon biologique déterminé, selon la Revendication 29, caractérisé en ce qu'il comprend les étapes de :
a) mise en contact de l'acide nucléique des mycobactéries éventuellement présent dans l'échantillon biologique testé, si nécessaire dans des conditions permettant l'accessibilité sous forme d'ADN simple brin avec au moins un couple d'amorces nucléotidiques selon l'une quelconque des Revendications 10 à 13 ;
b) séparation des brins d'ADN synthétisés, de leur matrice ;
c) répétition de la synthèse de produit d'élongation, à partir de chaque brin d'ADN présent à l'issue de l'étape b) et susceptible d'hybrider avec les amorces, jusqu'à l'obtention d'une amplification de l'ADN recherché, suffisante pour être détectée,
d) mise en contact du produit de l'étape c) avec une sonde nucléotidique dans des conditions permettant de détecter la présence du fragment d'ADN amplifié recherché;
e) détection des produits de l'hybridation éventuellement formés.

36. Procédé pour le diagnostic in vitro selon la Revendication 34, caractérisé en ce que la mise en contact de l'échantillon testé est précédée d'une étape de traitement de l'échantillon de façon à en extraire l'acide nucléique.

37. Procédé selon l'une quelconque des Revendications 35 ou 36, caractérisé en ce que préalablement à la mise en contact avec les amorces, on traite l'acide nucléique de l'échantillon avec une réverse-transcriptase, pour obtenir la synthèse d'ADNc à partir de l'ARN éventuellement présent dans l'échantillon testé.

38. Kit, coffret ou ensemble coordonné prêt à l'emploi pour la mise en oeuvre du procédé de détection d'au moins un groupe et/ou d'une espèce de mycobactéries selon l'une quelconque des Revendications 29 à 37, caractérisé en ce qu'il comprend, outre des quantités utiles de tampons et de réactifs appropriés pour la mise en oeuvre de ladite détection :
- des doses appropriées d'une paire d'amorces selon l'une quelconque des Revendications 10 à 13 ; et/ou
- des doses appropriées d'au moins une sonde ou un fragment de sonde nucléotidique selon l'une quelconque des Revendications 18, 21, 22 ou 23.

39. Kit pour le diagnostic in vitro d'une infection par des mycobactéries, sur un échantillon biologique déterminé, selon la Revendication 38, caractérisé en ce qu'il comprend :
- au moins un couple d'amorces nucléotidiques selon l'une quelconque des Revendications 10 à 13,
- des réactifs nécessaires à l'extraction des acides nucléiques à partir de l'échantillon traité,
- des réactifs pour effectuer la polymérisation dudit fragment d'ADN, à partir des amorces nucléotidiques, notamment des enzymes de polymérisation, en quantité suffisante pour réaliser l'amplification du fragment d'ADN que l'on souhaite amplifier,
- au moins un fragment nucléotidique pouvant être utilisé comme sonde et capable d'hybrider dans des conditions déterminées 16 à 18 h à 68°C ou 2 h à 65°C avec le fragment d'ADN amplifié,
- un contrôle interne de la réaction d'amplification, par exemple constitué par un fragment d'ADN éventuellement porté par un plasmide, ledit fragment pouvant être aisément détecté, par exemple en ce qu'il contient un gène de résistance à un antibiotique, ledit fragment étant en outre muni à ses deux extrémités, d'au moins une amorce d'amplification, de préférence choisie parmi les amorces de l'Invention,
- une sonde capable d'hybrider avec la fragment d'ADN contenu dans le contrôle interne,
- le cas échéant, une réverse-transcriptase pour obtenir de l'ADNc à partir d'ARN éventuellement présent dans l'échantillon testé,
- le cas échéant des moyens pour révéler l'hybridation.

40. Kit pour le diagnostic in vitro selon la Revendication 39, caractérisé en ce que les amorces nucléotidiques sont ISTB2 et ISTB7 et en ce qu'on utilise au moins une sonde de détection constituée par le fragment nucléotidique IS-2 marqué, éventuellement complétée par la sonde constituée par le fragment nucléotidique IS-6 marqué.

41. Séquence d'acide nucléique, spécifique du Mycobacterium tuberculosis ayant la séquence :

10          20          30          40          50          60
GTCGACACGC CTTCTGCACG GGAAGTCCTT CTGCGGCCAT CGTTGCTATG GCCGCTTACT
70          80          90          100         110         120
GCCTTCTAGT CCGTGCGGCT CTCGCAACAG CTCACGGGAC CTTTTTGAGG ATCGCCACTT
130         140         150         160         170         180
CAGGTCTTCA ACTCGCGGAT GCCCTCATTC GCAACGTTTG CGGCCTGCCT TGGGGCGGCC
190         200         210         220         230         240
GGCAGCCACC AAGTCGAGCA CTTTGCGGCG GAACTACTCG GGGTAACACT TCGGCACGGA
250         260         270         280         290         300
CACGGCTCGT TCGACGGACG TCGTGACCAG AAGTCGAGCA AACCGACTCC ACTCTAGCTA
310         320         330         340         350         360
GTGATACAAG CTTTTTTCTA GCCGCGCGAT GAACCCCCCC GGCATGTCCG GAGACTCCAG
370         380         390         400         410         420
TTCTTCGAAA GGATCGGGTC ATCTCAGGTG GTTCATCCAG GAGGTACCCG CCGGAGCTGC
430         440         450         460         470         480
GTGAGCGGGC GGTGCGGATG GTCGCAGAGA TCCGCGGTCA GCACGATTCG GAGTGGGCAG
490         500         510         520         530         540
CGATCAGTGA GGTCGCCCGT CTACTTGGTG TTGGCTGCGC GGAGACGGTG CGTAAGTGGG
550         560         570         580         590         600
TGCGCCAGGC GCAGGTCGAT GCCGGCGCAC GGCCCGGGAC CACGACCGAA GAATCCGCTG
610         620         630         640         650         660
AGCTGAAGCG CTTAGCGGCG GGACAACGCC GAATTGCCGA GGGCGAACGC GATTTTAAAG
670         680         690         700         710         720
ACCGCGTCGG CTTTCTTCGC GGCCGAGCTC GACCGGCCAG CACGCTAATT AACGGTTCAT
730         740         750         760         770         780
CGCCGATCAT CAGGGCCACC GCGAGGGCCC CGATGGTTTG CGGTGGGGTG TCGAGTCGAT

790 800 810 820 830 840
CTGCACACAG CTGACCGAGC TGGGTGTGCC GATCGCCCCA TCGACCTACT ACGACCACAT
850 860 870 880 890 900
GAACCGGGAG CCCAGCCGCC GCCAGCTGCG CGATGGCGAA CTCAAGGAGC ACATCAGCCC
910 920 930 940 950 960
CGTCCACGCC GCCAACTACC GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCCT CGGTCGGAGC GGTCGGAAGC TCCTATGACA ATGCACTAGC
1450 1460 1470 1480 1490 1500
CGAGACGATC AACGGCCTAT ACAAGACCGA GCTCATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

## Revendications (Revendications pour l'(les) Etat(s) contractant(s) suivant(s): ES)

1. Procédé d'obtention d'une séquence nucléotidique issue de mycobactéries, caractérisé en ce que l'on prépare une séquence nucléotidique répétée dans le génome d'une mycobactérie et spécifique du groupe du bacille de la tuberculose, qui s'hybride très fortement avec *M. tuberculosis,* c'est-à-dire que, lorsqu'elle est marquée au phosphore 32, elle fournit une tache visible à l'oeil nu après autoradiographie d'une heure et qui s'hybride très faiblement avec M. bovis BCG, c'est-à-dire que, lorsqu'elle est marquée au phosphore 32, elle fournit une tache visible à l'oeil nu après autoradiographie de 48 heures.

2. Procédé d'obtention d'une séquence selon la Revendication 1, caractérisé en ce que l'on prépare une séquence comportant à son extrémité 5' la séquence 5'TGAACCGCCCCGG 3' de formule I et à son extrémité 3' la séquence 5' CCGGGGCGGTTCA 3' de formule II, ladite séquence contenant en outre au moins un fragment d'une séquence de formule III suivante :

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TGGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTCTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
CTGCACACAG CTGACCCAGC TCGGTGTGCC GATCGCCCCA TCGACCTACT ACGACCACAT
850 860 870 880 890 900
GAACCGGGAG CCCAGCCGCC GCCAGCTGCC CGATGGCGAA CTCAAGGAGC ACATCAGCCC
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTCGGCT GGCGGGTCGC TTCCACGATG GCCACCTCCA TGGTCCTCGA
1270 1280 1290 1300 1310 1320
CGCGATCCAG CAAGCCATCT GGACCCGCCA ACAAGAAGGC GTACTCGACC TGAAAGACGT
1330 1340 1350 1360 1370 1380
TATCCACCAT ACCGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTCATCAAA CCCGGCAAGC CCTGGCGGTC
1510 1520 1530 1540 1550 1560
CATCGAGGAT GTCGAGTTCG CCACCGCCGC CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACC
1630 1640 1650 1660 1670 1680
CCAGAGACCA GCCGCCGGCT GAGGTCTCAG ATCAGAGAGT CTCCGGACTC ACCGGGGCGG

TTCA

Formule III

3. Procédé d'obtention d'une séquence nucléotidique selon la Revendication 2, caractérisé en ce que l'on prépare une séquence comprenant le fragment 343-1152 de la séquence de formule III selon la Revendication 2 et toute séquence présentant au moins 80 % d'homologie avec celle-ci.

4. Procédé d'obtention d'une séquence nucléotidique selon la Revendication 2, caractérisé en ce que l'on prépare une séquence comprenant le fragment 327-1684 de la séquence de formule III selon la Revendication 2 et toute séquence présentant au moins 80 % d'homologie avec celle-ci.

5. Procédé d'obtention d'un oligonucléotide, caractérisé en ce que l'on prépare un fragment d'une séquence nucléotidique selon l'une quelconque des Revendications 1 à 4.

6. Procédé d'obtention d'un oligonucléotide selon la Revendication 5, caractérisé en ce que l'on prépare l'oligonucléotide de formule IV suivante :

ATGTCAGGTGGTTCATCGAG (IV).

7. Procédé d'obtention d'un oligonucléotide selon la Revendication 5, caractérisé en ce que l'on prépare l'oligonucléotide de formule V suivante :

ACAGGCCGAGTTTGGTCATC (V).

8. Procédé d'obtention de fragments nucléotidiques selon la Revendication 5, caractérisé en ce que l'on prépare un fragment présentant l'une des séquences suivantes :

ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)
IS-2 : 5' CATCAGCCGCGTCCACGCCGCCAACTACGG 3' (XIV)
IS-4 : 5' TGTGCCGATCGCCCCATCGACCTACTACGA 3' (XV)
IS-6 : 5' GTCGAGTCGATCTGCACACAGCTGACCGAG 3 ' (XVI)

9. Procédé d'obtention de fragments nucléotidiques, caractérisé en ce que l'on prépare un fragment complémentaire des fragments selon la Revendication 8 ou des fragments modifiés par rapport aux précédents, par enlèvement ou addition de nucléotide(s) dans une proportion d'environ 10 à 15 % par rapport à la longueur des fragments ci-dessus et/ou par modification de la nature des nucléotides, dès lors que les fragments nucléotidiques modifiés conservent une capacité d'hybridation avec la séquence d'ADN de mycobactéries, analogue à celle que présentent les fragments correspondants non modifiés.

10. Procédé d'obtention d'amorces pour l'amplification de fragments d'ADN de mycobactéries, notamment pour l'amplification de fragments d'une séquence de Mycobacterium tuberculosis, caractérisé en ce que l'on prépare une séquence ou un fragment de séquence tel que défini dans l'une quelconque des Revendications 6 à 9, choisis parmi les suivants ou parmi leurs fragments nucléotidiques complémentaires :

ISTB1 : 5' ATGTCAGGTGGTTCATCGAG 3'
ISTB2 : 5' ACAGGCCGAGTTTGGTCATC 3'
ISTB3 : 5' TTCGCAATTCTGGCGTTGTCC 3'
ISTB4 : 5' TCGACCTACTACGACCACAT 3'
ISTB5 : 5' GTCGAGTCGATCTGCACACA 3'
ISTB6 : 5' GTTCAGGGTTAGCCACACTT 3'
ISTB7 : 5' CAGCACGCTAATTAACCCGG 3'
ISTB8 : 5' TGGCCAGATGCACCGTCGAA 3'
ISTB9 : 5' AGTACACATCGATCCGGTTC 3'
ISTB10 : 5' TACTACGCTCAACGCCAGAG 3'

11. Procédé d'obtention d'amorces pour l'amplification de fragments d'ADN de mycobactéries, selon la Revendication 10, caractérisé en ce que l'on prépare les paires d'amorces suivantes :

ISTB2 et ISTB4 :
5' ACAGGCCGAGTTTGGTCATC 3' et
5' TCGACCTACTACGACCACAT 3'
ISTB2 et ISTB5 :
5' ACAGGCCGAGTTTGGTCATC 3' et
5' GTCGAGTCGATCTGCACACA 3'
ISTB5 et ISTB6 :
5' GTCGAGTCGATCTGCACACA 3' et
5' GTTCAGGGTTAGCCACACTT 3'
ISTB6 et ISTB7 :
5' GTTCAGGGTTAGCCACACTT 3' et
5' CAGCACGCTAATTAACCCGG 3'.

12. Procédé d'obtention d'amorces pour l'amplification de fragments d'ADN selon la Revendication 10 ou la Revendication 11, caractérisé en ce que l'on prépare le couple d'amorces suivant :

ISTB2 et ISTB7 :

5' ACAGGCCGAGTTTGGTCATC 3' et
5' CAGCACGCTAATTAACCCGG 3'.

13. Procédé d'obtention d'amorces selon l'une quelconque des Revendications 10 à 12, caractérisé en ce que l'on marque lesdites amorces par un marqueur chimique, physique ou enzymatique et/ou en ce que l'on fixe lesdites amorces à un support solide, notamment un support particulaire ou membranaire, en particulier des billes magnétiques.

14. Procédé d'obtention de produits de traduction et/ou fragments de ceux-ci, caractérisé en ce que lesdits produits sont préparés à partir d'une séquence ou d'un fragment de séquence nucléotidique selon l'une quelconque des revendications 1 à 4.

15. Procédé d'obtention de sonde nucléique apte à détecter par hybridation la présence d'ADN spécifique du Mycobacterium tuberculosis dans un échantillon biologique, caractérisé en ce que l'on prépare une sonde présentant une séquence ayant une longueur d'au moins 20 bases, capable de s'hybrider, après mise en contact pendant 16 à 18 h à 68°C ou 2 h à 65°C, avec une partie de la séquence constituée par le fragment 327-1684 selon la Revendication 4, se situant entre les deux amorces nucléiques.

16. Procédé d'obtention d'une sonde selon la Revendication 15, caractérisé en ce que l'on prépare l'un des fragments nucléotidiques suivants :

IS-2 : 5' CATCAGCCGCGTCCACGCCGCCAACTACGG 3'
IS-4 : 5' TGTGCCGATCGCCCCATCGACCTACTACGA 3'
IS-6 : 5' GTCGAGTCGATCTGCACACAGCTGACCGAG 3'

17. Procédé d'obtention d'une sonde pour la détection de fragments d'ADN amplifiés de mycobactéries, selon la Revendication 15, caractérisé en ce que l'on prépare des fragments nucléotidiques spécifiques d'une séquence de mycobactérie, selon l'une quelconque des Revendications 5 à 8, capables d'hybrider avec ledit fragment d'ADN amplifié après contact pendant 16 à 18 h à 68°C ou 2 h à 65°C, lesdits fragments étant soit marqués à leur extrémité 5' et/ou 3' par une substance que l'on peut détecter, par exemple par un isotope radioactif, une enzyme, un marqueur chimique ou chimioluminescent approprié, un fluorochrome, un haptène, ou un anticorps, des analogues de bases ou encore un marqueur physique, soit fixés à un support solide notamment un support particulaire ou membranaire, par exemple des billes magnétiques.

18. Procédé de détection d'une séquence répétée spécifique du groupe du bacille de la tuberculose, selon l'une quelconque des Revendications 1 à 4, caractérisé en ce qu'il comprend :
(1) une étape dans laquelle on réalise une banque de cosmides recombinants comprenant des fragments d'ADN d'une mycobactérie appropriée supérieurs à 30 kb ; et
(2) une étape dans laquelle on détecte le/les clones cosmidiques contenant au moins une séquence répétée, par hybridation 16 à 18 h à 68°C avec un ADN génomique total de mycobactérie appropriée, marqué de manière convenable.

19. Procédé d'obtention d'un réactif de diagnostic pour la détection d'au moins un groupe de mycobactéries, caractérisé en ce que l'on prépare ledit réactif à partir d'une séquence nucléotidique ou un fragment de celle-ci selon l'une quelconque des Revendications 1 à 8 pour la détection des mycobactéries du groupe du bacille de la tuberculose, éventuellement associée à au moins une autre séquence nucléotidique appropriée à la détection d'un autre groupe de mycobactéries et/ou à au moins un marqueur approprié.

20. Procédé d'obtention d'un réactif selon la Revendication 19, caractérisé en ce que l'on prépare des paires d'amorces pour la synthèse d'un fragment d'ADN ou d'ARN du groupe du bacille de la tuberculose, chaque amorce comprenant une séquence ou un fragment de séquence nucléotidique selon l'une quelconque des Revendications 1 à 8.

21. Procédé d'obtention d'un réactif selon la Revendication 20, caractérisé en ce que l'on prépare une paire d'amorces comprenant des oligonucléotides appariés conformément aux Revendications 10 à 13.

22. Procédé d'obtention d'un réactif selon la Revendication 19, caractérisé en ce que l'on prépare une sonde de détection appropriée d'un fragment d'ADN ou d'ARN du groupe du bacille de la tuberculose.

23. Procédé d'obtention d'un réactif selon la Revendication 22, caractérisé en ce que l'on prépare une séquence nucléotidique de formule III, selon l'une quelconque des Revendications 2 à 4.

24. Procédé d'obtention d'un réactif selon la Revendication 19 ou la Revendication 22, caractérisé en ce que le marqueur est choisi dans le groupe qui comprend notamment les isotopes radioactifs, les enzymes appropriées, les fluorochromes, les marqueurs chimiques appropriés, les haptènes et les anticorps ou les analogues de base appropriées.

25. Procédé d'obtention d'une famille de plasmides recombinants, caractérisés en ce que l'on insère une séquence nucléotidique selon l'une quelconque des Revendications 1 à 4 dans un plasmide approprié.

26. Procédé d'obtention d'un plasmide selon la Revendication 25, caractérisé en ce que l'on insère la séquence nucléotidique de formule III selon la Revendication 2 ou un fragment de celle-ci ou une séquence homologue dans un plasmide approprié.

27. Procédé d'obtention d'un plasmide selon la Revendication 25, caractérisé en ce que ladite séquence associée à un vecteur pUC18.

28. Procédé d'obtention d'un plasmide selon la Revendication 26 ou la Revendication 27, caractérisé en ce que ledit plasmide, dénommé pMT01, a été déposé sous le n°I-900, en date du 25 août 1989, auprès de la Collection Nationale de Cultures de Microorganismes tenue par l'Institut Pasteur.

29. Procédé de détection et d'identification rapide d'au moins un groupe et/ou une espèce de mycobactéries dans un échantillon biologique, caractérisé en ce qu'il comprend :
(1) une étape dans laquelle l'on met en contact l'échantillon biologique avec au moins un réactif de diagnostic selon l'une quelconque des Revendications 20 à 24,
(2) une étape dans laquelle on détecte par tout moyen approprié le ou les produits résultant de l'interaction séquence nucléotidique de mycobactérie éventuellement présente-réactif de diagnostic.

30. Procédé selon la Revendication 29, caractérisé en ce que le/les réactifs de diagnostic de l'étape (1) sont une/des paires d'amorces selon l'une quelconque des revendications 10 à 13 et permettent l'obtention de produits d'amplification de la séquence nucléotidique à détecter.

31. Procédé selon la Revendication 30, caractérisé en ce que les produits d'amplification obtenus en (1) sont détectés par séparation électrophorétique.

32. Procédé selon la Revendication 30, caractérisé en ce que les produits d'amplification obtenus en (1) sont détectés par hybridation 2 h à 65°C entre lesdits produits d'amplification et un réactif de diagnostic selon l'une quelconque des Revendications 19, 22, 23 et 24.

33. Procédé selon la Revendication 29, caractérisé en ce que le réactif de diagnostic de l'étape (1) est une sonde de détection selon l'une quelconque des Revendications 19, 22, 23 ou 24.

34. Procédé de détection de la présence de Mycobacterium tuberculosis dans un échantillon biologique, selon la Revendication 29, caractérisé par les étapes suivantes :
i) mise en contact de l'échantillon biologique avec un couple de fragments d'acide nucléiques, dits amorces, selon l'une quelconque des Revendications 10 à 13, l'ADN contenu dans l'échantillon ayant été, le cas échéant, préalablement rendu accessible à l'hybridation et dans des conditions permettant une hybridation des amorces à l'ADN de Mycobacterium tuberculosis, telles que 2 min à 60°C ;
ii) amplification de l'ADN de Mycobacterium tuberculosis ;
iii) mise en évidence de l'amplification de fragments d'ADN correspondant au fragment encadré par les amorces, notamment par électrophorèse sur gel ;
iv) vérification éventuelle de la séquence du fragment amplifié, notamment par hybridation de sonde spécifique, par séquençage ou par analyse de site de restriction.

35. Procédé pour le diagnostic in vitro d'une infection par des mycobactéries sur un échantillon biologique déterminé, selon la Revendication 29, caractérisé en ce qu'il comprend les étapes de :
a) mise en contact de l'acide nucléique des mycobactéries éventuellement présent dans l'échantillon biologique testé, si nécessaire dans des conditions permettant l'accessibilité sous forme d'ADN simple brin avec au moins un couple d'amorces nucléotidiques selon l'une quelconque des Revendications 10 à 13 ;
b) séparation des brins d'ADN synthétisés, de leur matrice ;
c) répétition de la synthèse de produit d'élongation, à partir de chaque brin d'ADN présent à l'issue de l'étape b) et susceptible d'hybrider avec les amorces, jusqu'à l'obtention d'une amplification de l'ADN recherché, suffisante pour être détectée,
d) mise en contact du produit de l'étape c) avec une sonde nucléotidique dans des conditions permettant de détecter la présence du fragment d'ADN amplifié recherché ;
e) détection des produits de l'hybridation éventuellement formés.

36. Procédé pour le diagnostic in vitro selon la Revendication 34, caractérisé en ce que la mise en contact de l'échantillon testé est précédée d'une étape de traitement de l'échantillon de façon à en extraire l'acide nucléique.

37. Procédé selon l'une quelconque des Revendications 35 ou 36, caractérisé en ce que préalablement à la mise en contact avec les amorces, on traite l'acide nucléique de l'échantillon avec une réverse-transcriptase, pour obtenir la synthèse d'ADNc à partir de l'ARN éventuellement présent dans l'échantillon testé.

38. Kit, coffret ou ensemble coordonné prêt à l'emploi pour la mise en oeuvre du procédé de détection d'au moins un groupe et/ou d'une espèce de mycobactéries selon l'une quelconque des Revendications 29 à 37, caractérisé en ce qu'il comprend, outre des quantités utiles de tampons et de réactifs appropriés pour la mise en oeuvre de ladite détection :
- des doses appropriées d'une paire d'amorces selon l'une quelconque des Revendications 10 à 13 ; et/ou
- des doses appropriées d'au moins une sonde ou un fragment de sonde nucléotidique selon l'une quelconque des Revendications 18, 21, 22 ou 23.

39. Kit pour le diagnostic in vitro d'une infection par des mycobactéries, sur un échantillon biologique déterminé, selon la Revendication 38, caractérisé en ce qu'il comprend :
- au moins un couple d'amorces nucléotidiques selon l'une quelconque des Revendications 10 à 13,
- des réactifs nécessaires à l'extraction des acides nucléiques à partir de l'échantillon traité,
- des réactifs pour effectuer la polymérisation dudit fragment d'ADN, à partir des amorces nucléotidiques, notamment des enzymes de polymérisation, en quantité suffisante pour réaliser l'amplification du fragment d'ADN que l'on souhaite amplifier,
- au moins un fragment nucléotidique pouvant être utilisé comme sonde et capable d'hybrider dans des conditions déterminées 16 à 18 h à 68°C ou 2 h à 65°C avec le fragment d'ADN amplifié,
- un contrôle interne de la réaction d'amplification, par exemple constitué par un fragment d'ADN éventuellement porté par un plasmide, ledit fragment pouvant être aisément détecté, par exemple en ce qu'il contient un gène de résistance à un antibiotique, ledit fragment étant en outre muni à ses deux extrémités, d'au moins une amorce d'amplification, de préférence choisie parmi les amorces de l'Invention,
- une sonde capable d'hybrider avec la fragment d'ADN contenu dans le contrôle interne,
- le cas échéant, une réverse-transcriptase pour obtenir de l'ADNc à partir d'ARN éventuellement présent dans l'échantillon testé, - le cas échéant des moyens pour révéler l'hybridation.

40. Kit pour le diagnostic in vitro selon la Revendication 39, caractérisé en ce que les amorces nucléotidiques sont ISTB2 et ISTB7 et en ce qu'on utilise au moins une sonde de détection constituée par le fragment nucléotidique IS-2 marqué, éventuellement complétée par la sonde constituée par le fragment nucléotidique IS-6 marqué.

41. Procédé d'obtention d'une séquence d'acide nucléique, spécifique du Mycobacterium tuberculosis, caractérisé en ce que l'on prépare une séquence de formule suivante :

10 20 30 40 50 60
GTCGACACGC CTTCTGCACG GGAAGTCCTT CTGCGGCCAT CGTTGCTATG GCCGGTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTGA ACTCGCGGAT GCCCTCATTG GCAACGTTTG CGGCCTGCCT TGGGGGGGCC
190 200 210 220 230 240
GGCAGCCACC AAGTCGAGGA CTTTCCGGCG GAACTACTCC GGGTAACACT TCGGCACGGA
250 260 270 280 290 300
CACGGCTCGT TCGACGGACG TCGTGACCAG AAGTCGAGGA AACCGACTCC ACTCTAGCTA
310 320 330 340 350 360
GTGATACAAG CTTTTTTCTA GCCGCGCGAT GAACCCCCCC GGCATGTCCG GAGACTCCAG
370 380 390 400 410 420
TTCTTCGAAA GGATCGGGTC ATCTCAGGTG GTTCATCCAG GAGGTACCCG CCGGAGCTGC
430 440 450 460 470 480
GTGAGCCGGC CGTGCGGATG GTCGCAGAGA TCCGCGGTCA GCACGATTCG GAGTGGGCAG
490 500 510 520 530 540
CGATCAGTGA GGTCGCCCGT CTACTTCGTG TTCGCTGCGC GGAGACGGTG CCTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCCAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
[?]TGCACACAG CTGACCGAGC TCGGTGTGCC GATCGCCCCA TCGACCTACT ACGACCACAT
850 860 870 880 890 900
GAACCGGGAG CCCAGCCGCC GCGAGCTGCG CGATGGCGAA CTCAAGGAGC ACATCAGCCG
910 920 930 940 950 960
CGTCCACGCC GCCAACTACG GTGTTTACGG TGCCGGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACCATC AACGGCCTAT ACAAGACCGA GCTGATCAAA CCCGGCAAGC CCTGGCGGTC
1510 1520 1530 1540 1550 1560
CATCGAGGAT GTCGAGTTCG CCACCGCCGG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCCCCGGCT GAGGTCTCAG ATCAGAGAGT CTCCGGACTC ACCGGGGCGG

TTCA

## Patentansprüche (Patentansprüche für folgende(n) Vertragsstaat(en): AT, BE, CH, DE, DK, FR, GB, IT, LI, LU, NL, SE)

1. Nucleotidsequenz aus Mykobakterien, dadurch **gekennzeichnet**, daß sie aus einer in dem Genom eines Mykobakteriums repetitiven und für die Gruppe der Tuberkulosebakterien spezifischen Nucleotidsequenz besteht und daß sie sehr stark mit M. tuberculosis hybridisiert, d.h. wenn sie mit Phosphor-32 markiert wird, einen mit dem unbewaffneten Auge nach einstündiger Autoradiographie sichtbaren Fleck liefert und daß sie sehr schwach mit M. bovis BCG hybridisiert, d.h. wenn sie mit Phosphor-32 markiert wird, einen mit dem unbewaffneten Auge nach 48stündiger Autoradiographie sichtbaren Fleck liefert.

2. Sequenz nach Anspruch 1, dadurch **gekennzeichnet**, daß sie an ihrem 5'-Ende die Sequenz 5'-TGAACCGCCCCGG-3' der Formel I und an ihrem 3'-Ende die Sequenz 5'-CCGGGGCGGTTCA-3' der Formel II besitzt, wobei die Sequenz weiterhin mindestens ein Fragment einer Sequenz der nachstehenden Formel III enthält:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC

430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT

730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TGGGTGTGCC GATCGCCCCA TCGACCTACT ACGACCACAT
850 860 870 880 890 900
CAACCGGGAG CCCAGCCGCC GCGAGCTGCG CGATGGCGAA CTCAAGGAGC ACATCAGCCG
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT

1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

FORMEL III

3. Nucleotidsequenz nach Anspruch 2, dadurch **gekennzeichnet,** daß sie das Fragment 343-1152 der Sequenz der Formel III nach Anspruch 2 und jede Sequenz, die eine mindestens achtzigprozentige Homologie damit besitzt, umfaßt.

4. Nucleotidsequenz nach Anspruch 2, dadurch **gekennzeichnet,** daß sie das Fragment 327-1684 der Sequenz der Formel III nach Anspruch 2 und jede Sequenz, die eine mindestens achtzigprozentige Homologie damit besitzt, umfaßt.

5. Oligonucleotid, dadurch **gekennzeichnet**, daß es aus einem Fragment einer Nucleotidsequenz nach einem der Ansprüche 1 bis 4 besteht.

6. Oligonucleotid nach Anspruch 5, dadurch **gekennzeichnet,** daß es die Sequenz der nachstehenden Formel IV besitzt:

ATGTCAGGTGGTTCATCGAG (IV).

7. Oligonucleotid nach Anspruch 5, dadurch **gekennzeichnet,** daß es die Sequenz der nachstehenden Formel V besitzt:

ACAGGCCGAGTTTGGTCATC (V).

8. Nucleotidfragmente nach Anspruch 5, dadurch **gekenneichnet,** daß sie eine der nachstehenden Sequenzen besitzen:

ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)
IS-2 : 5' CATCAGCCGCGTCCACGCCGCCAACTACGG 3' (XIV)
IS-4 : 5' TGTGCCGATCGCCCCATCGACCTACTACGA 3' (XV)
IS-6 : 5' GTCGAGTCGATCTGCACACAGCTGACCGAG 3' (XVI)

9. Nucleotidfragmente, dadurch **gekennzeichnet,** daß es sich um Fragmente, die den Fragmenten nach Anspruch 8 komplementär sind, oder um Fragmente, die bezüglich der vorstehend genannten durch Entfernen oder Hinzufügen eines Nucleotids bzw. von Nucleotiden in einem Verhältnis von etwa 10 bis 15 %, bezogen auf die Länge der vorstehenden Fragmente und/oder durch Modifikation der Natur der Nucleotide modifiziert wurden, handelt, wobei die modifizierten Nucleotidfragmente eine Hybridisierungsfähigkeit mit der DNA-Sequenz der Mykobakterien beibehalten, die derjenigen analog ist, die die entsprechenden nichtmodifizierten Fragmente besitzen.

10. Startersequenzen für die Amplifikation von DNA-Fragmenten von Mykobakterien, insbesondere für die Amplifikation von Fragmenten einer Sequenz von Mycobacterium tuberculosis, dadurch **gekennzeichnet**, daß sie Nucleotidfragmenten nach einem der Ansprüche 6 bis 9, ausgewählt aus den folgenden oder aus ihren komplementären Nucleotidfragmenten, entsprechen:

ISTB1 : 5' ATGTCAGGTGGTTCATCGAG 3'
ISTB2 : 5' ACAGGCCGAGTTTGGTCATC 3'
ISTB3 : 5' TTCGCAATTCTGGCGTTGTCC 3'
ISTB4 : 5' TCGACCTACTACGACCACAT 3'
ISTB5 : 5' GTCGAGTCGATCTGCACACA 3'
ISTB6 : 5' GTTCAGGGTTAGCCACACTT 3'
ISTB7 : 5' CAGCACGCTAATTAACCCGG 3'
ISTB8 : 5' TGGCCAGATGCACCGTCGAA 3'
ISTB9 : 5' AGTACACATCGATCCGGTTC 3'
ISTB10 : 5' TACTACGCTCAACGCCAGAG 3'

11. Startersequenzen für die Amplifikation von-DNA-Fragmenten von Mykobakterien nach Anspruch 10, dadurch **gekennzeichnet,** daß sie in Zweierkombinationen verwendet werden und daß es sich um die folgenden Paare handelt:

ISTB2 und ISTB4:
5' ACAGGCCGAGTTTGGTCATC 3' und
5' TCGACCTACTACGACCACAT 3'
ISTB2 und ISTB5:
5' ACAGGCCGAGTTTGGTCATC 3' und
5' GTCGAGTCGATCTGCACACA 3'
ISTB5 und ISTB6:
5' GTCGAGTCGATCTGCACACA 3' und
5' GTTCAGGGTTAGCCACACTT 3'
ISTB6 und ISTB7:
5' GTTCAGGGTTAGCCACACTT 3' und
5' CAGCACGCTAATTAACCCGG 3'.

12. Startersequenzen für die Amplifikation von DNA-Fragmenten nach Anspruch 10 oder 11, dadurch **gekennzeichnet,** daß sie dem Paar der folgenden Startersequenzen entsprechen:

ISTB2 und ISTB7:
5' ACAGGCCGAGTTTGGTCATC 3' und
5' CAGCACGCTAATTAACCCGG 3'.

13. Startersequenzen nach einem der Ansprüche 10 bis 12, dadurch **gekennzeichnet**, daß sie mit einem chemischen, physikalischen oder enzymatischen Marker markiert sind und/oder daß sie an einen festen Träger, insbesondere an einem teilchenförmigen oder membranartigen Träger, insbesondere an Magnetkügelchen, gebunden sind.

14. Translationsprodukte und/oder Fragmente davon, dadurch **gekennzeichnet**, daß sie von einer Sequenz oder einem Fragment der Nucleotidsequenz nach einem der Ansprüche 1 bis 4 codiert werden.

15. Nucleinsäuresonde mit der Eignung zum Nachweis der Anwesenheit von für Mycobacterium tuberculosis spezifischer DNA durch Hybridisation in einer biologischen Probe, dadurch **gekennzeichnet** daß sie eine Sequenz mit einer Länge von mindestens 20 Basen umfaßt, die nach einem 16- bis 18stündigen Inkontaktbringen bei 68°C oder einem zweistündigen Inkontaktbringen bei 65°C mit einem Teil der Sequenz hybridisieren können, die aus dem Fragment 327-1684 nach Anspruch 4 besteht, die zwischen den zwei Nucleinsäurestartersequenzen angeordnet sind.

16. Sonde nach Anspruch 15, dadurch **gekennzeichnet,** daß es sich um eines der nachstehenden Nucleotidfragmente handelt:

IS-2 : 5' CATCAGCCGCGTCCACGCCGCCAACTACGG 3'
IS-4 : 5' TGTGCCGATCGCCCCATCGACCTACTACGA 3'
IS-6 : 5' GTCGAGTCGATCTGCACACAGCTGACCGAG 3'

17. Sonden zum Nachweis von amplifizierten DNA-Fragmenten von Mykobakterien nach Anspruch 15, dadurch **gekennzeichnet,** daß es sich um Nucleotidfragmente, die für eine Mykobakteriensequenz spezifisch sind, nach einem der Ansprüche 5 bis 8 handelt, die mit dem amplifizierten DNA-Fragment nach Kontakt von 16 bis 18 Stunden bei 68°C oder 2 Stunden *bei* 65°C hybridisieren können, wobei die Fragmente entweder an ihrem 5'- und/oder 3'-Ende durch eine nachweisbare Substanz markiert sind, beispielsweise durch ein radioaktives Isotop, ein Enzym, einen geeigneten chemischen oder chemilumineszierenden Marker, ein Fluorochrom, ein Hapten oder einen Antikörper, Basenanaloga oder auch einen physikalischen Marker oder an einem festen Träger, insbesondere an einem teilchenförmigen oder membranartigen Träger, beispielsweise Magnetkügelchen, gebunden sind.

18. Verfahren zum Nachweis einer repetitiven, für die Gruppe der Tuberkulosebakterien spezifischen Sequenz-nach einem der Ansprüche 1 bis 4, dadurch **gekennzeichnet,** daß man
(1) in einer Stufe eine Bibliothek aus rekombinanten Cosmiden, umfassend mehr als 30 kb lange DNA-Fragmente eines geeigneten Mykobakteriums anlegt; und
(2) in einer Stufe den/die Cosmidclon/Cosmidclone, der/die mindestens eine repetitive Sequenz enthält/enthalten durch Hybridisierung von 16 bis 18 Stunden bei 68°C mit einer gesamten genomischen DNA des geeigneten Mykobakteriums, die in geeigneter Weise markiert ist, nachweist.

19. Diagnostisches Reagens zum Nachweis von mindestens einer Gruppe von Mykobakterien, dadurch **gekenn zeichnet**, daß es mindestens eine Nucleotidsequenz oder ein Fragment davon nach einem der Ansprüche 1 bis 8 zum Nachweis von Mykobakterien der Gruppe der Tuberkulosebakterien, gegebenenfalls zusammen mit mindestens einer anderen geeigneten Nucleotidsequenz, zum Nachweis einer anderen Gruppe von Mykobakterien und/oder mindestens einem geeigneten Marker umfaßt.

20. Reagens nach Anspruch 19, dadurch **gekennzeichnet**, daß es Startersequenzpaare für die Synthese eines DNA- oder RNA-Fragments der Gruppe der Tuberkulosebakterien umfaßt, wobei jede Startersequenz eine Sequenz oder ein Fragment der Nucleotidsequenz nach einem der Ansprüche 1 bis 8 umfaßt.

21. Reagens nach Anspruch 20, dadurch **gekennzeichnet,** daß es aus einem Startersequenzpaar, umfassend gepaarte Oligonucleotide nach den Ansprüchen 10 bis 13, umfaßt.

22. Reagens nach Anspruch 19, dadurch **gekennzeichnet**, daß es eine geeignete Nachweissonde für ein DNA- oder RNA-Fragment der Gruppe der Tuberkulosebakterien umfaßt.

23. Reagens nach Anspruch 22, dadurch **gekenn-zeichnet**, daß die Nachweissonde vorteilhafterweise eine Nucleotidsequenz der Formel III nach einem der Ansprüche 2 bis 4 umfaßt.

24. Reagens nach Anspruch 19 oder 22, dadurch **ge kennzeichnet,** daß der Marker aus der Gruppe umfassend insbesondere radioaktive Isotope, geeignete Enzyme, Fluorochrome, geeignete chemische Marker, Haptene und Antikörper oder geeignete Basenanaloga ausgewählt ist.

25. Familie von rekombinanten Plasmiden, dadurch **ge kennzeichnet**, daß sie mindestens eine Nucleotidsequenz nach einem der Ansprüche 1 bis 4 enthält.

26. Plasmid nach Anspruch 25, dadurch **gekennzeichnet,** daß es die Nucleotidsequenz der Formel III nach Anspruch 2 oder ein Fragment davon oder eine homologe Sequenz umfaßt.

27. Plasmid nach Anspruch 25, dadurch **gekennzeichnet**, daß es die Sequenz in Assoziation mit einem pUC18-Vektor umfaßt.

28. Plasmid nach Anspruch 26 oder 27, dadurch **gekennzeichnet**, daß es als pMT01 bezeichnet wurde und daß es unter der Hinterlegungsnummer I-900 am 25. August 1989 bei der Collection Nationale de Cultures de Microorganismes des Pasteur-Instituts hinterlegt wurde.

29. Verfahren zum raschen Nachweis und zur raschen Identifikation mindestens einer Gruppe und/oder einer Art von Mykobakterien in einer biologischen Probe, dadurch **ge kennzeichnet**, daß man
(1) in einer Stufe die biologische Probe mit mindestens einem diagnostischen Reagens nach einem der Ansprüche 20 bis 24 in Kontakt bringt,
(2) in einer Stufe durch jedes geeignete Mittel das oder die Produkte, das bzw. die aus der Wechselwirkung der gegebenenfalls vorhandenen Nucleotidsequenz des Mykobakteriums mit dem diagnostischen Reagens hervorgeht bzw. hervorgehen, nachweist.

30. Verfahren nach Anspruch 29, dadurch **gekennzeichnet,** daß das/die diagnostischen Reagentien der Stufe (1) ein Startersequenzpaar/-sequenzpaare nach einem der Ansprüche 10 bis 13 sind und den Erhalt von Amplifikationsprodukten der nachzuweisenden Nucleotidsequenz erlauben.

31. Verfahren nach Anspruch 30, dadurch **gekennzeichnet,** daß man die in (1) erhaltenen Amplifikationsprodukte durch elektrophoretische Trennung nachweist.

32. Verfahren nach Anspruch 30, dadurch **gekennzeichnet**, daß man die in (1) erhaltenen Amplifikationsprodukte durch zweistündige Hybridisierung bei 65°C zwischen den Amplifikationsprodukten und einem diagnostischen Reagens nach einem der Ansprüche 19, 22, 23 und 24 nachweist.

33. Verfahren nach Anspruch 29, dadurch **gekennzeichnet,** daß das diagnostische Reagens der Stufe (1) eine Nachweissonde nach einem der Ansprüche 19, 22, 23 oder 24 ist.

34. Verfahren zum Nachweis der Anwesenheit von Mycobacterium tuberculosis in einer biologischen Probe nach Anspruch 29, dadurch **gekennzeichnet**, daß man in Stufen
i) die biologische Probe mit einem Paar von Nucleinsäurefragmenten, sogenannten Startersequenzen, nach einem der Ansprüche 10 bis 13 in Kontakt bringt, wobei die in der Probe enthaltene DNA gegebenenfalls vorher der Hybridisierung zugänglich gemacht wurde und unter Bedingungen, die eine Hybridisierung der Startersequenzen mit der DNA von Mycobacterium tuberculosis erlauben, für zwei Minuten bei 60°C;
ii) die DNA von Mycobacterium tuberculosis amplifiziert;
iii) die Amplifikation der dem durch die Startersequenzen eingeschlossenen DNA-Fragmenten insbesondere gelelektrophoretisch nachweist;
iv) gegebenenfalls die Sequenz des amplifizierten Fragments insbesondere durch Hybridisierung der spezifischen Sonde durch Sequenzierung oder durch Restriktionsstellenanalyse bestätigt.

35. Verfahren zur in vitro Diagnose einer Infektion durch Mykobakterien an einer bestimmten biologischen Probe nach Anspruch 29, dadurch **gekennzeichnet,** daß man in Stufen
a) die Nucleinsäure der gegebenenfalls in der getesteten biologischen Probe vorhandenen Mykobakterien, sofern notwendig, unter Bedingungen, die die Zugänglichkeit in Form einzelsträngiger DNA mit mindestens einem Paar Nucleotidstartersequenzen nach einem der Ansprüche 10 bis 13 erlauben, in Kontakt bringt;
b) die synthetisierten DNA-Stränge von ihrer Matrix trennt;
c) die Synthese des Elongationsprodukts aus jedem DNA-Strang, der am Ende von Stufe b) vorhanden ist und mit den Startersequenzen hybridisieren kann, wiederholt, bis eine Amplifikation der gewünschten DNA erhalten ist, die ausreichend, um nachgewiesen werden zu können;
d) das Produkt von Stufe c) mit einer Nucleotidsonde unter Bedingungen, die den Nachweis der Anwesenheit des gewünschten amplifizierten DNA-Fragments erlauben, in Kontakt bringt;
e) die gegebenenfalls gebildeten Hybridisierungsprodukte nachweist.

36. Verfahren zur in vitro Diagnose nach Anspruch 34, dadurch **gekennzeichnet**, daß man die getestete Probe nach einer derartigen Behandlungsstufe der Probe, daß daraus die Nucleinsäure extrahiert wird, in Kontakt bringt.

37. Verfahren nach einem der Ansprüche 35 oder 36, dadurch **gekennzeichnet**, daß man vor dem Inkontaktbringen mit den Startersequenzen die Nucleinsäure der Probe mit einer reversen Transkriptase behandelt, um die Synthese von cDNA aus der gegebenenfalls in der getesteten Probe vorhandenen RNA zu erhalten.

38. Gebrauchsfertiges Kit, Reagenzzusammenstellung oder Reagenzbesteck zur Durchführung des Nachweisverfahrens auf mindestens eine Gruppe und/oder eine Art von Mykobakterien nach einem der Ansprüche 29 bis 37, dadurch **gekennzeichnet,** daß es außer nützlichen Mengen von Puffern und geeigneten Reagentien zur Durchführung des Nachweises
- geeignete Konzentrationen eines Startersequenzpaares nach einem der Ansprüche 10 bis 13; und/oder
- geeignete Mengen mindestens einer Sonde oder eines Fragments der Nucleotidsonde nach einem der Ansprüche 18, 21, 22 oder 23 umfaßt.

39. Kit zur in vitro Diagnose einer Infektion durch Mykobakterien an einer bestimmten biologischen Probe nach Anspruch 38, dadurch **gekennzeichnet**, daß es umfaßt:
- mindestens ein Paar Nucleotidstartersequenzen nach einem der Ansprüche 10 bis 13,
- Reagentien, die zur Extraktion von Nucleinsäuren aus der behandelten Probe notwendig sind,
- Reagentien zur Durchführung der Polymerisation des DNA-Fragments aus Nucleotidstartersequenzen, insbesondere Polymerisationsenzyme, in ausreichender Menge zur Durchführung der Amplifikation des DNA-Fragments, das man amplifizieren möchte,
- mindestens ein Nucleotidfragment, das als Sonde verwendet werden kann, und das unter bestimmten Bedingungen, 16 bis 18 Stunden bei 68°C oder 2 Stunden bei 65°C, mit dem amplifizierten DNA-Fragment hybridisieren kann,
- eine interne Kontrolle der Amplifikationsreaktion, beispielsweise bestehend aus einem, gegebenenfalls von einem Plasmid getragenen, DNA-Fragment, wobei das Fragment leicht nachgewiesen werden kann, beispielsweise dadurch, daß es ein Antibiotikumresistenzgen enthält, wobei das Fragment weiterhin an seinen zwei Enden mindestens eine Amplifikationsstartersequenz, bevorzugt ausgewählt aus den erfindungsgemäßen Startersequenzen, enthält,
- eine Sonde, die mit dem DNA-Fragment, das in der internen Kontrolle enthalten ist, hybridisieren kann,
- gegebenenfalls eine reverse Transkriptase, um cDNA aus gegebenenfalls in der getesteten Probe vorhandenen RNA zu erhalten,
- gegebenenfalls Mittel zum Nachweis der Hybridisierung.

40. Kit zur in vitro Diagnose nach Anspruch 39, dadurch **gekennzeichnet**, daß die Nucleotidstartersequenzen ISTB2 und ISTB7 sind und daß man mindestens eine Nachweissonde, bestehend aus dem markierten Nucleotidfragment IS-2, gegebenenfalls komplettiert durch die Sonde, die aus dem markierten Nucleotidfragment IS-6 besteht, verwendet.

41. Nucleinsäuresequenz, die für Mycobacterium tuberculosis spezifisch ist, mit der Sequenz:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGAC[?] C[?]TTTTGAGG ATCGCCACTT
130 140 150 160 170 180
CAGGTCTTCA ACTCGCGGAT GCCGTCATTG GCAACGTTTG CGCCCTGCCT TGGGGCGGCC
190 200 210 220 230 240
GGCAGCCACC AAGTCGAGCA CTTTGCGGCG GAACTACTCG GGTAACACT[?] TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTCTA GCCGCCGGAT GAACCGCCGC GGCATGTCCG GAGACTCCAG
370 380 390 400 410 420
TTCTTCGAAA GGATGGGTC[?] ATGTCAGGTG GTTCATCCAG GAGGTACCCG CCGGAGCTGC
430 440 450 460 470 480
GTCAGCGGGC GGTGCCGATG GTCGCAGAGA TCCGCGGTCA GCACGATTCG GAGTGGGCAG
490 500 510 520 530 540
CGATCAGTCA GGTCGCCCGT CTACTTCGTG TTGGCTGCGC GGAGACGGTG CCTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCCAA GGGCGAACGC GATTTTAAAG
670 680 690 700 710 720
ACCCCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TCGGTGTGCG GATCGCCGCA TCGACCTACT ACGACCACAT
850 860 870 880 890 900
GAACCGGGAG CCGAGCCGCC GCGAGCTGCG CGATGGCCAA CTCAAGGAGC ACATCAGCCG
910 920 930 940 950 960
CGTCGACGCC GCCAACTACC GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC

1030 1040 1050 1060 1070 1080
CGGGACCACC CGCGGCAAAG CCCGCAGGAC CACGATCGCT GATCGGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCGTACGTG GCGTTTGTCA CCGACGCCTA
1210 1220 1230 1240 1250 1260
CGCTCGCAGG ATCGTCGGCT GGCGGGTCGC TTCGACGATG GCGACCTCGA TGGTCGTCGA
1270 1280 1290 1300 1310 1320
CGCGATCGAG CAAGCCATCT GGACCCGGCA ACAAGAAGGC GTACTCGACC TCAAAGACGT
1330 1340 1350 1360 1370 1380
TATCGACCAT ACGGATAGGG GATCTCAGTA CACATCGATC CGGTTCAGCG AGCGGCTCGC
1390 1400 1410 1420 1430 1440
CGAGGCAGGC ATCGAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTCGCGGTC
1510 1520 1530 1540 1550 1560
CATCGAGGAT GTCGAGTTCG CCACCGCCGC CTGGTCGCAC TGGTTCAACC ATCGCCGGCT
1570 1580 1590 1600 1610 1620
CTACGAGTAC TGCGGCCACC TCCCCCCGGT CGAACTCGAG GCTGCCTACT ACGCTCAACG
1630 1640 1650 1660 1670 1680
CCAGAGACCA GCCCCCCGCT GAGGTCTCAG ATCAGAGAGT CTCCGGACTC ACCGGGGCGG

TTGA

## Patentansprüche (Patentansprüche für folgende(n) Vertragsstaat(en): ES)

1. Verfahren zur Herstellung einer Nucleotidsequenz aus Mykobakterien, dadurch **gekennzeichnet**, daß man eine in dem Genom eines Mykobakteriums repetitive und für das Genom der Tuberkulosebakterien spezifische Nucleotidsequenz herstellt, die sehr stark mit M. tuberculosis hybridisiert, d.h. wenn sie mit Phosphor-32 markiert wird, einen mit dem unbewaffneten Auge nach einstündiger Autoradiographie sichtbaren Fleck liefert und die sehr schwach mit M. bovis BCG hybridisiert, d.h. wenn sie mit Phosphor-32 markiert wird, einen mit dem unbewaffneten Auge nach 18stündiger Autoradiographie sichtbaren Fleck liefert.

2. Verfahren zur Herstellung einer Sequenz nach Anspruch 1, dadurch **gekennzeichnet**, daß man eine Sequenz herstellt, die an ihrem 5'-Ende die Sequenz 5'-TGAACCGCCCCGG-3' der Formel I und an ihrem 3'-Ende die Sequenz 5'-CCGGGGCGGTTCA-3' der Formel II trägt, wobei die Sequenz weiterhin mindestens ein Fragment einer Sequenz der nachstehenden Formel III enthält:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG

370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT
790 800 810 820 830 840
CTGCACACAG CTGACCGAGC TGGGTGTGCC GATCGCCCCA TCGACCTACT ACGACCACAT
850 860 870 880 890 900
CAACCGGGAG CCCAGCCGCC GCGAGCTGCG CGATGGCGAA CTCAAGGAGC ACATCAGCCG
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCGACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT

1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

FORMEL III

3. Verfahren zur Herstellung einer Nucleotidsequenz nach Anspruch 2, dadurch **gekennzeichnet**, daß man eine Sequenz, umfassend das Fragment 343-1152 der Sequenz der Formel III nach Anspruch 2 und jede Sequenz, die-mindestens eine achtzigprozentige Homologie damit besitzt, herstellt.

4. Verfahren zur Herstellung einer Nucleotidsequenz nach Anspruch 2, dadurch **gekennzeichnet**, daß man eine Sequenz, umfassend das Fragment 327-1684 der Sequenz der Formel III nach Anspruch 2 und jede Sequenz, die mindestens eine achtzigprozentige Homologie damit besitzt, herstellt.

5. Verfahren zur Herstellung eines Oligonucleotids, dadurch **gekennzeichnet,** daß man ein Fragment einer Nucleotidsequenz nach einem der Ansprüche 1 bis 4 herstellt.

6. Verfahren zur Herstellung eines Oligonucleotids nach Anspruch 5, dadurch **gekennzeichnet**, daß man das Oligonucleotid der nachstehenden Formel IV herstellt:

ATGTCAGGTGGTTCATCGAG (IV).

7. Verfahren zur Herstellung eines Oligonucleotids nach Anspruch 5, dadurch **gekennzeichnet,** daß man das Oligonucleotid der nachstehenden Formel V herstellt:

ACAGGCCGAGTTTGGTCATC (V).

8. Verfahren zur Herstellung von Nucleotidfragmenten nach Anspruch 5, dadurch **gekennzeichnet**, daß man ein Fragment mit einer der nachstehenden Sequenzen herstellt:

ISTB-3 : 5' TTCGCAATTCTGGCGTTGTCC 3' (VI)
ISTB-4 : 5' TCGACCTACTACGACCACAT 3' (VII)
ISTB-5 : 5' GTCGAGTCGATCTGCACACA 3' (VIII)
ISTB-6 : 5' GTTCAGGGTTAGCCACACTT 3' (IX)
ISTB-7 : 5' CAGCACGCTAATTAACCCGG 3' (X)
ISTB-8 : 5' TGGCCAGATGCACCGTCGAA 3' (XI)
ISTB-9 : 5' AGTACACATCGATCCGGTTC 3' (XII)
ISTB-10 : 5' TACTACGCTCAACGCCAGAG 3' (XIII)
IS-2 : 5' CATCAGCCGCGTCCACGCCGCCAACTACGG 3' (XIV)
IS-4 : 5' TGTGCCGATCGCCCCATCGACCTACTACGA 3' (XV)
IS-6 : 5' GTCGAGTCGATCTGCACACAGCTGACCGAG 3 ' (XVI)

9. Verfahren zur Herstellung von Nucleotidfragmenten, dadurch **gekennzeichnet,** daß man ein den Fragmenten nach Anspruch 8 oder bezüglich der vorstehenden Sequenzen durch Entfernen oder Hinzufügen eines Nucleotids bzw. von Nucleotiden in einem Verhältnis von *ungefähr* 10 bis 15%, bezogen auf die Länge der vorstehenden Fragmente und/oder durch Modifikation der Natur der Nukleotide modifizierten Fragmente komplementäres Fragment herstellt, wobei die modifizierten Nucleotidfragmente eine Hybridisierungsfähigkeit mit der DNA-Sequenz von Mykobakterien beibehalten, die derjenigen analog ist, die entsprechende nichtmodifizierte Fragmente zeigen.

10. Verfahren zur Herstellung von Startersequenzen für die Amplifikation von DNA-Fragmenten von Mykobakterien, insbesondere für die Amplifikation von Fragmenten einer Sequenz von Mycobacterium tuberculosis, dadurch **gekennzeichnet**, daß man eine Sequenz oder ein Sequenzfragment, wie in einem der Ansprüche 6 bis 9 definiert, ausgewählt aus den folgenden oder aus ihren komplementären Nucleotidfragmenten, herstellt:

ISTB1 : 5' ATGTCAGGTGGTTCATCGAG 3'
ISTB2 : 5' ACAGGCCGAGTTTGGTCATC 3'
ISTB3 : 5' TTCGCAATTCTGGCGTTGTCC 3'
ISTB4 : 5' TCGACCTACTACGACCACAT 3'
ISTB5 : 5' GTCGAGTCGATCTGCACACA 3'
ISTB6 : 5' GTTCAGGGTTAGCCACACTT 3'
ISTB7 : 5' CAGCACGCTAATTAACCCGG 3'
ISTB8 : 5' TGGCCAGATGCACCGTCGAA 3'
ISTB9 : 5' AGTACACATCGATCCGGTTC 3'
ISTB10 : 5' TACTACGCTCAACGCCAGAG 3'

11. Verfahren zur Herstellung von Startersequenzen zur Amplifikation von DNA-Fragmenten von Mykobakterien nach Anspruch 10, dadurch **gekennzeichnet**, daß man die folgenden Startersequenzpaare herstellt:

ISTB2 und ISTB4:
5' ACAGGCCGAGTTTGGTCATC 3' und
5' TCGACCTACTACGACCACAT 3'
ISTB2 und ISTB5:
5' ACAGGCCGAGTTTGGTCATC 3' und
5' GTCGAGTCGATCTGCACACA 3'
ISTB5 und ISTB6:
5' GTCGAGTCGATCTGCACACA 3' und
5' GTTCAGGGTTAGCCACACTT 3'
ISTB6 und ISTB7:
5' GTTCAGGGTTAGCCACACTT 3' und
5' CAGCACGCTAATTAACCCGG 3'.

12. Verfahren zur Herstellung von Startersequenzen für die Amplifikation von DNA-Fragmenten nach Anspruch 10 oder 11, dadurch **gekennzeichnet**, daß man das folgende Paar von Startersequenzen herstellt:

ISTB2 und ISTB7:
5' ACAGGCCGAGTTTGGTCATC 3' und
5' CAGCACGCTAATTAACCCGG 3'.

13. Verfahren zur Herstellung von Startersequenzen nach einem der Ansprüche 10 bis 12, dadurch **gekennzeichnet**, daß man die Startersequenzen mit einem chemischen, physikalischen oder enzymatischen Marker markiert und/oder daß man die Startersequenzen an einen festen Träger, insbesondere einem teilchenförmigen oder membranartigen Träger, insbesondere Magnetkügelchen, bindet.

14. Verfahren zur Herstellung von Translationsprodukten und/oder Fragmenten davon, dadurch **gekenzeichnet**, daß man die Produkte aus einer Sequenz oder einem Nucleotidsequenzfragment nach einem der Ansprüche 1 bis 4 herstellt.

15. Verfahren zur Herstellung einer Nucleinsäuresonde mit der Eignung, durch Hybridisieren die Anwesenheit von DNA, die für Mycobacterium tuberculosis spezifisch ist, in einer biologischen Probe nachzuweisen, dadurch **gekennzeichnet**, daß man eine Sonde herstellt, die eine Sequenz mit einer Länge von mindestens 20 Basen besitzt, die nach Inkontaktbringen während 16 bis 18 Stunden bei 68°C oder 2 Stunden bei 65°C mit einem Teil der Sequenz hybridisieren kann, die aus dem Fragment 327-1684 nach Anspruch 4 besteht, die zwischen den zwei Nucleinsäurestartersequenzen angeordnet ist.

16. Verfahren zur Herstellung einer Sonde nach Anspruch 15, dadurch **gekennzeichnet**, daß man eines der nachstehenden Nucleotidfragmente herstellt:

IS-2 : 5' CATCAGCCGCGTCCACGCCGCCAACTACGG 3'
IS-4 : 5' TGTGCCGATCGCCCCATCGACCTACTACGA 3'
IS-6 : 5' GTCGAGTCGATCTGCACACAGCTGACCGAG 3'

17. Verfahren zur Herstellung einer Sonde zum Nachweis von amplifizierten DNA-Fragmenten von Mykobakterien nach Anspruch 15, dadurch **gekennzeichnet**, daß man für eine Sequenz des Mykobakteriums spezifische Nucleotidfragmente nach einem der Ansprüche 5 bis 8 herstellt, die mit dem amplifizierten DNA-Fragment nach Kontakt während 16 bis 18 Stunden bei 68°C oder 2 Stunden bei 65°C hybridisieren können, wobei die Fragmente entweder an ihrem 5'- und/oder 3'-Ende durch eine nachweisbare Substanz markiert sind, beispielsweise durch ein radioaktives Isotop, ein Enzym, einen geeigneten chemischen oder chemilumineszierenden Marker, ein Fluorochrom, ein Hapten oder einen Antikörper, Basenanaloga oder auch einen physikalischen Marker oder an einem festen Träger, insbesondere an einem teilchenförmigen oder membranartigen Träger, beispielsweise Magnetkügelchen, fixiert sind.

18. Verfahren zum Nachweis einer repetitiven, für die Gruppe der Tuberkulosebakterien spezifischen Sequenz nach einem der Ansprüche 1 bis 4, dadurch **gekennzeichnet**, daß man
(1) in einer Stufe eine Bibliothek aus rekombinanten Cosmiden, umfassend mehr als 30 kb lange DNA-Fragmente eines geeigneten Mykobakteriums anlegt; und
(2) in einer Stufe den/die Cosmidclon/Cosmidclone, der/die mindestens eine repetitive Sequenz enthält/enthalten, durch Hybridisierung von 16 bis 18 Stunden bei 68°C mit einer gesamten genomischen DNA des geeigneten Mykobakteriums, die in geeigneter Weise markiert ist, nachweist.

19. Verfahren zur Herstellung eines diagnostischen Reagens zum Nachweis von mindestens einer Gruppe von Mykobakterien, dadurch **gekennzeichnet,** daß man das Reagens aus einer Nucleotidsequenz oder einem Fragment davon nach einem der Ansprüche 1 bis 8 zum Nachweis von Mykobakterien der Gruppe der Tuberkulosebakterien, gegebenenfalls zusammen mit mindestens einer anderen für die Detektion einer anderen Gruppe von Mykobakterien geeigneten Nucleotidsequenz und/oder mindestens einem geeigneten Marker herstellt.

20. Verfahren zur Herstellung eines Reagens nach Anspruch 19, dadurch **gekennzeichnet**, daß man Startersequenzpaare für die Synthese eines DNA- oder RNA-Fragments der Gruppe der Tuberkulosebakterien herstellt, wobei jede Startersequenz eine Sequenz oder ein Fragment der Nucleotidsequenz nach einem der Ansprüche 1 bis 8 umfaßt.

21. Verfahren zur Herstellung eines Reagens nach Anspruch 20, dadurch **gekennzeichnet**, daß man ein Startersequenzpaar, umfassend gepaarte Oligonucleotide nach den Ansprüchen 10 bis 13, herstellt.

22. Verfahren zur Herstellung eines Reagens nach Anspruch 19, dadurch **gekennzeichnet**, daß man eine Sonde, die für die Detektion eines DNA- oder RNA-Fragments der Gruppe der Tuberkulosebakterien geeignet ist, herstellt.

23. Verfahren zur Herstellung eines Reagens nach Anspruch 22, dadurch **gekennzeichnet,** daß man eine Nucleotidsequenz der Formel III nach einem der Ansprüche 2 bis 4 herstellt.

24. Verfahren zur Herstellung eines Reagens nach Anspruch 19 oder 22, dadurch **gekennzeichnet,** daß man den Marker aus der Gruppe umfassend insbesondere radioaktive Isotope, geeignete Enzyme, Fluorochrome, geeignete chemische Marker, Haptene und Antikörper oder geeignete Basenanaloga auswählt.

25. Verfahren zur Herstellung einer Familie von rekombinanten Plasmiden, dadurch **gekennzeichnet**, daß man eine Nucleotidsequenz nach einem der Ansprüche 1 bis 4 in ein geeignetes Plasmid insertiert.

26. Verfahren zur Herstellung eines Plasmids nach Anspruch 25, dadurch **gekennzeichnet**, daß man die Nucleotidsequenz der Formel III nach Anspruch 2 oder ein Fragment davon oder eine homologe Sequenz in ein geeignetes Plasmid insertiert.

27. Verfahren zur Herstellung eines Plasmids nach Anspruch 25, dadurch **gekennzeichnet**, daß man die Sequenz mit einem pUC18-Vektor assoziiert.

28. Verfahren zur Herstellung eines Plasmids nach Anspruch 26 oder 27, dadurch **gekennzeichnet**, daß das Plasmid mit der Bezeichnung pMT01 unter der Hinterlegungsnummer I-900 am 25. August 1989 bei der Collection Nationale de Cultures de Microorganismes des Pasteur-Instituts hinterlegt wurde.

29. Verfahren zum raschen Nachweis und zur raschen Identifikation mindestens einer Gruppe und/oder einer Art von Myobakterien in einer biologischen Probe, dadurch **gekennzeichnet**, daß man
(1) in einer Stufe die biologische Probe mit mindestens einem diagnostischen Reagens nach einem der Ansprüche 20 bis 24 in Kontakt bringt,
(2) in einer Stufe durch jedes geeignete Mittel das oder die Produkte, das bzw. die aus der Wechselwirkung der gegebenenfalls vorhandenen Nucleotidsequenz des Mykobakteriums mit dem diagnostischen Reagens hervorgeht bzw. hervorgehen, nachweist.

30. Verfahren nach Anspruch 29, dadurch **gekennzeichnet**, daß das/die diagnostischen Reagentien der Stufe (1) ein Startersequenzpaar/-sequenzpaare nach einem der Ansprüche 10 bis 13 sind und den Erhalt von Amplifikationsprodukten der nachzuweisenden Nucleotidsequenz erlauben.

31. Verfahren nach Anspruch 30, dadurch **gekennzeichnet**, daß man die in (1) erhaltenen Amplifikationsprodukte durch elektrophoretische Trennung nachweist.

32. Verfahren nach Anspruch 30, dadurch **gekennzeichnet**, daß man die in (1) erhaltenen Amplifikationsprodukte durch zweistündige Hybridisierung bei 65°C zwischen den Amplifikationsprodukten und einem diagnostischen Reagens nach einem der Ansprüche 19, 22, 23 und 24 nachweist.

33. Verfahren nach Anspruch 29, dadurch **gekennzeichnet**, daß das diagnostische Reagens der Stufe (1) eine Nachweissonde nach einem der Ansprüche 19, 22, 23 oder 24 ist.

34. Verfahren zum Nachweis der Anwesenheit von Mycobacterium tuberculosis in einer biologischen Probe nach Anspruch 29, dadurch **gekennzeichnet**, daß man in Stufen
i) die biologische Probe mit einem Paar von Nucleinsäurefragmenten, sogenannten Startersequenzen, nach einem der Ansprüche 10 bis 13 in Kontakt bringt, wobei die in der Probe enthaltene DNA gegebenenfalls vorher der Hybridisierung zugänglich gemacht wurde und unter Bedingungen, die eine Hybridisierung der DNA-Startersequenzen von Mycobacterium tuberculosis erlauben, für zwei Minuten bei 60°C;
ii) die DNA von Mycobacterium tuberculosis amplifiziert;
iii) die Amplifikation der dem durch die Startersequenzen eingeschlossenen DNA-Fragmenten insbesondere gelelektrophoretisch nachweist;
iv) gegebenenfalls die Sequenz des amplifizierten Fragments insbesondere durch Hybridisierung der spezifischen Sonde durch Sequenzierung oder durch Restriktionsstellenanalyse bestätigt.

35. Verfahren zur in vitro Diagnose einer Infektion durch Mykobakterien an einer bestimmten biologischen Probe nach Anspruch 29, dadurch **gekennzeichnet**, daß man in Stufen
a) die Nucleinsäure der gegebenenfalls in der getesteten biologischen Probe vorhandenen Mykobakterien, sofern notwendig, unter Bedingungen, die die Zugänglichkeit in Form einzelsträngiger DNA mit mindestens einem Paar Nucleotidstartersequenzen nach einem der Ansprüche 10 bis 13 erlauben, in Kontakt bringt;
b) die synthetisierten DNA-Stränge von ihrer Matrix trennt;
c) die Synthese des Elongationsprodukts aus jedem DNAStrang*,* der am Ende von Stufe b) vorhanden ist und mit den Startersequenzen hybridisieren kann, wiederholt, bis eine Amplifikation der gewünschten DNA erhalten ist, die ausreicht, um nachgewiesen werden zu können;
d) das Produkt von Stufe c) mit einer Nucleotidsonde unter Bedingungen, die den Nachweis der Anwesenheit des gewünschten amplifizierten DNA-Fragments erlauben, in Kontakt bringt;
e) die gegebenenfalls gebildeten Hybridisierungsprodukte nachweist.

36. Verfahren zur in vitro Diagnose nach Anspruch 34, dadurch **gekennzeichnet**, daß man die getestete Probe nach einer derartigen Behandlungsstufe der Probe, daß daraus die Nucleinsäure extrahiert wird, in Kontakt bringt.

37. Verfahren nach einem der Ansprüche 35 oder 36, dadurch **gekennzeichnet**, daß man vor dem Inkontaktbringen mit den Startersequenzen die Nucleinsäure der Probe mit einer reversen Transkriptase behandelt, um die Synthese von cDNA aus der gegebenenfalls in der getesteten Probe vorhandenen RNA zu erhalten.

38. Gebrauchsfertiges Kit, Reagenzzusammenstellung oder Reagenzbesteck zur Durchführung des Nachweisverfahrens auf mindestens eine Gruppe und/oder eine Art von Mykobakterien nach einem der Ansprüche 29 bis 37, dadurch **gekennzeichnet**, daß es außer nützlichen Mengen von Puffern und geeigneten Reagentien zur Durchführung des Nachweises
- geeignete Konzentrationen eines Startersequenzpaares nach einem der Ansprüche 10 bis 13; und/oder
- geeignete Mengen mindestens einer Sonde oder eines Fragments der Nucleotidsonde nach einem der Ansprüche 18, 21, 22 oder 23 umfaßt.

39. Kit zur in vitro Diagnose einer Infektion durch Mykobakterien an einer bestimmten biologischen Probe nach Anspruch 38, dadurch **gekennzeichnet**, daß es umfaßt:
- mindestens ein Paar Nucleotidstartersequenzen nach einem der Ansprüche 10 bis 13,
- Reagentien, die zur Extraktion von Nucleinsäuren aus der behandelten Probe notwendig sind,
- Reagentien zur Durchführung der Polymerisation des DNA-Fragments aus Nucleotidstartersequenzen, insbesondere Polymerisationsenzyme, in ausreichender Menge zur Durchführung der Amplifikation des DNA-Fragments, das man amplifizieren möchte,
- mindestens ein Nucleotidfragment, das als Sonde verwendet werden kann, und das unter bestimmten Bedingungen, 16 bis 18 Stunden bei 68°C oder 2 Stunden bei 65°C, mit dem amplifizierten DNA-Fragment hybridisieren kann,
- eine interne Kontrolle der Amplifikationsreaktion, beispielsweise bestehend aus einem, gegebenenfalls von einem Plasmid getragenen, DNA-Fragment, wobei das Fragment leicht nachgewiesen werden kann, beispielsweise dadurch, daß es ein Antibiotikumresistenzgen enthält, wobei das Fragment weiterhin an seinen zwei Enden mindestens eine Amplifikationsstartersequenz, bevorzugt ausgewählt aus den erfindungsgemäßen Startersequenzen, enthält,
- eine Sonde, die mit dem DNA-Fragment, das in der internen Kontrolle enthalten ist, hybridisieren kann,
- gegebenenfalls eine reverse Transkriptase, um cDNA aus gegebenenfalls in der getesteten Probe vorhandenen RNA zu erhalten,
- gegebenenfalls Mittel zum Nachweis der Hybridisierung.

40. Kit zur in vitro Diagnose nach Anspruch 39, dadurch **gekennzeichnet**, daß die Nucleotidstartersequenzen ISTB2 und ISTB7 sind und daß man mindestens eine Nachweissonde, bestehend aus dem markierten Nucleotidfragment IS-2, gegebenenfalls komplettiert durch die Sonde, die aus dem markierten Nucleotidfragment IS-6 besteht, verwendet.

41. Verfahren zur Herstellung einer für Mycobacterium tuberculosis spezifischen Nucleinsäuresequenz, dadurch **gekennzeichnet**, daß man eine Sequenz der nachstehenden Formel herstellt:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGGCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCCACGGACG TCGTGACCAG AAGTCGAGCA AACCGACTCC ACTCTAGCTA
310 320 330 340 350 360
GTGATACAAG CTTTTTTCTA GCCGCGCGAT GAACCGCCCC GGCATGTCCG GAGACTCCAG
370 380 390 400 410 420
TTCTTCGAAA GGATCGGGTC ATCTCAGGTG GTTCATCGAG GAGGTACCCG CCGGAGCTGC
430 440 450 460 470 480
GTGAGCGGGC GGTGCGGATG GTCGCAGAGA TCCCGGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGCG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
[?]TGCACACAG CTGACCCAGC TCGGTCTGCC GATCGCCCCA TCGACCTACT ACGACCACAT
850 860 870 880 890 900
GAACCGGGAG CCCAGCCGCC CCCAGCTGCG CGATGGCCAA CTCAAGGAGC ACATCAGCCG
910 920 930 940 950 960
CGTCCACGCC GCCAACTACG GTGTTTACGG TGCCGGCAAA GTGTGGCTAA CCCTCAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCCCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GGTGATCAAA CCCGGCAAGC CCTGGCGGTC
1510 1520 1530 1540 1550 1560
GATCGAGGAT GTCGAGTTCG CCACCCGCCG CTGGGTCGAC TGGTTCAACC ATCCCCGCCT
1570 1580 1590 1600 1610 1620
CTACCAGTAC TGCGGCCACG TCCCGCCGGT CGAACTCGAG GCTGCCTACT ACGCTCAACG
1630 1640 1650 1660 1670 1680
CCAGAGACCA GCCCCCGGCT GAGGTCTCAG ATCAGAGAGT CTCCGGACTC ACCGGGGCGG

TTCA

## Claims (Claims for the following Contracting State(s): AT, BE, CH, DE, DK, FR, GB, IT, LI, LU, NL, SE)

1. A nucleotide sequence derived from mycobacteria, characterised in that it is constituted by a nucleotide sequence repeated in the genome of a mycobacterium and specific to the group of tuberculosis bacilli and in that it hybridises very strongly with *M.tuberculosis,* that is to say when it is marked with phosphorous 32 it provides a spot visible to the naked eye after autoradiography for one hour and in that it hybridises very weakly with M.bovis BCG, that is to say when it is marked with phosphorous 32 it supplies a spot visible to the naked eye after autoradiography for 48 hours.

2. A sequence according to claim 1, characterised in that, at its end 5', it comprises the sequence 5'TGAACCGCCCCGG 3' of formula I while at its end 3' it comprises the sequence 5'CCGGGGCGGTTCA 3' of formula II, the said sequence containing additionally at least one fragment of a sequence of the following formula III:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
CTGCACACAG CTCACCCAGC TCGGTGTGCC GATCGCCCCA TCGACCTACT ACGACCACAT
850 860 870 880 890 900
GAACCGGGAG CCCAGCCGCC GCCAGCTGCG CGATGGCGAA CTCAAGGAGC ACATCAGCCC
910 920 930 940 950 960
CGTCGACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCGCAGGAC CACGATCGCT GATCCGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTCGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCCT CCGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCCAGTTCG CCACCCGCCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCGCCGGCT GAGGTCTCAG ATCAGAGAGT CTCCGGACTC ACCGGGGCGG

TTCA

3. A nucleotide sequence according to claim 2, characterised in that it comprises the fragment 343-1152 of the sequence of formula III in accordance with claim 2 and any sequence displaying at least 80% homology therewith.

4. A nucleotide sequence according to claim 2, characterised in that it comprises the fragment 327-1684 of the sequence of formula III in accordance with claim 2 and any sequence displaying at least 80% homology therewith.

5. An oligonucleotide, characterised in that it is constituted by a fragment of a nucleotide sequence according to any one of claims 1 to 4.

6. An oligonucleotide according to claim 5, characterised in that it displays the following sequence of formula IV:

ATGTCAGGTGGTTCATCGAG (IV)

7. An oligonucleotide according to claim 5, characterised in that it displays the following sequence of formula V:

ACAGGCCGAGTTTGGTCATC (V)

8. Nucleotide fragments according to claim 5, characterised in that they display one of the following sequences:

ISTB-3 : 5'TTCGCAATTCTGGCGATTGTCC 3' (VI)
ISTB-4 : 5'TCGACCTACTACGACCACAT 3' (VII)
ISTB-5 : 5'GTCGAGTCGATCTGCACACA 3' (VIII)
ISTB-6 : 5'GTTCAGGGTTAGCCACACTT 3' (IX)
ISTB-7 : 5'CAGCACGCTAATTAACCCGG 3' (X)
ISTB-8 : 5'TGGCCAGATGCACCGTCGAA 3' (XI)
ISTB-9 : 5'AGTACACATCGATCCGGTTC 3' (XII)
ISTB-10: 5'TACTACGCTCAACGCCAGAG 3' (XIII)
IS-2 : 5'CATCAGCCGCGTCCACGCCGCCAACTACGG 3' (XIV)
IS-4 : 5'TGTGCCGATCGCCCCATCGACCTACTACGA 3' (XV)
IS-6 : 5'GTCGAGTCGATCTGCACACAGCTGACCGAG 3' (XVI)

9. Nucleotide fragments characterised in that they are complementary fragments of the fragments according to claim 8 or fragments modified in comparison with the preceding fragments by removal or addition of nucleotide(s) in a proportion of approximately 10 to 15% in relation to the length of the above fragments and/or by modification of the nature of the nucleotides and hence the modified nucleotide fragments retain a capacity for hybridisation with the DNA sequence of mycobacteria similar to that displayed by the corresponding non-modified fragments.

10. Primers for amplifying DNA fragments of mycobacteria, particularly for amplifying fragments of a sequence of Mycobacterium tuberculosis, characterised in that they correspond to nucleotide fragments in accordance with any one of claims 6 to 9, chosen from the following or from their complementary nucleotide fragments:

ISTB1 : 5'ATGTCAGGTGGTTCATCGAG 3'
ISTB2 : 5'ACAGGCCGAGTTTGGTCATC 3'
ISTB3 : 5'TTCGCAATTCTGGCGTTGTCC 3'
ISTB4 : 5'TCGACCTACTACGACCACAT 3'
ISTB5 : 5'GTCGAGTCGATCTGCACACA 3'
ISTB6 : 5'GTTCAGGGTTAGCCACACTT 3'
ISTB7 : 5'CAGCACGCTAATTAACCCGG 3'
ISTB8 : 5'TGGCCAGATGCACCGTCGAA 3'
ISTB9 : 5'AGTACACATCGATCCGGTTC 3'
ISTB10: 5'TACTACGCTCAACGCCAGAG 3'

11. Primers for amplifying DNA fragments of mycobacteria in accordance with claim 10, characterised in that they are taken in combination, two by two, and in that they are the following pairings:

ISTB2 and ISTB4:
5'ACAGGCCGAGTTTGGTCATC 3' and
5'TCGACCTACTACGACCACAT 3'

ISTB2 and ISTB5:
5'ACAGGCCGAGTTTGGTCATC 3' and
5'GTCGAGTCGATCTGCACACA 3'
ISTB5 and ISTB6:
5'GTCGAGTCGATCTGCACACA 3' and
5'GTTCAGGGTTAGCCACACTT 3'
ISTB6 and ISTB7:
5'GTTCAGGGTTAGCCACACTT 3' and
5'CAGCACGCTAATTAACCCGG 3'.

12. Primers for amplifying DNA fragments according to claim 10 or claim 11, characterised in that they correspond to the following pair of initiators:

ISTB2 and ISTB7:
5'ACAGGCCGAGTTTGGTCATC 3' and
5'CAGCACGCTAATTAACCCGG 3'.

13. Primers according to any one of claims 10 to 12, characterised in that they are marked by a chemical, physical or enzymatic marker and/or in that they are fixed on a solid carrier, especially a particulate or membrane carrier, in particular magnetic balls.

14. Products of translation and/or fragments thereof, characterised in that they are coded by a sequence or a fragment of a nucleotide sequence according to any one of claims 1 to 4.

15. A nucleic probe capable of detecting by hybridisation the presence of DNA specific to Mycobacterium tuberculosis in a biological specimen, characterised in that it comprises a sequence having a length of at least 20 bases, capable of hybridising, after being brought into contact for 16 to 18 hours at 68°C or for 2 hours at 65°, with a part of the sequence constituted by the fragment 327-1684 in accordance with claim 4, and situated between the two nucleic primers.

16. A probe according to claim 15, characterised in that it is one of the following nucleotide fragments:

IS-2 : 5'CATCAGCCGCGTCCACGCCGCCAACTACGG 3'
IS-4 : 5'TGTGCCGATCGCCCCATCGACCTACTACGA 3'
IS-6 : 5'GTCGAGTCGATCTGCACACAGCTGACCGAG 3'.

17. Probes for detecting amplified DNA fragments of mycobacteria according to claim 15, characterised in that they are nucleotide fragments specific to a mycobacterium sequence according to any one of claims 5 to 8, capable of hybridising with the said amplified DNA fragment after contact for 16 to 18 hours at 68°C or for 2 hours at 65°C, the said fragments being either marked at their end 5' and/or 3' by a substance which is possible to detect, for example by a radioactive isotope, an enzyme, a suitable chemical or chemico-luminescent marker, a fluorochrome, a hapten or an antibody, analogues of bases or even a physical marker, or fixed to a solid carrier, especially a particulate or membrane type of carrier, for example magnetic balls.

18. A method of detecting a repeated sequence specific to the group of the tuberculosis bacillus according to any one of claims 1 to 4, characterised in that it comprises:
(1) a stage in which a recombining cosmide bank is prepared comprising fragments of DNA of a suitable mycobacterium in excess of 30 kb, and
(2) a stage in which the cosmide clones containing at least one repeated sequence is/are detected by hybridisation for 16 to 18 hours at 68°c with a total genome DNA of a suitable mycobacterium marked in an appropriate manner.

19. A diagnostic reagent for detecting at least one group of mycobacteria, characterised in that it comprises at least one nucleotide sequence or a fragment thereof in accordance with any one of claims 1 to 8 for detecting mycobacteria of the tuberculosis bacillus group, possibly associated with at least one other nucleotide sequence suitable for detecting another group of mycobacteria and/or with at least one suitable marker.

20. A reagent according to claim 19, characterised in that it comprises pairs of primers for the synthesis of a fragment of DNA or RNA of the tuberculosis bacillus group, each primer comprising a sequence or a fragment of a nucleotide sequence according to any one of claims 1 to 8.

21. A reagent according to claim 20, characterised in that it is constituted by a pair of primers comprising oligonucleotides paired in accordance with claims 10 to 13.

22. A reagent according to claim 19, characterised in that it comprises a suitable probe for detecting a fragment of DNA or RNA of the tuberculosis bacillus group.

23. A reagent according to claim 22, characterised in that the said detection probe advantageously comprises a nucleotide sequence of formula III according to any one of claims 2 to 4.

24. A reagent according to claim 19 or claim 22, characterised in that the marker is chosen from the group comprising in particular the radioactive isotopes, the suitable enzymes, the fluorochromes, the suitable chemical markers, the haptens and the antibodies or the suitable base analogues.

25. Family of recombining plasmids, characterised in that they contain at least one nucleotide sequence according to any one of claims 1 to 4.

26. A plasmid according to claim 25, characterised in that it comprises the nucleotide sequence of formula III in accordance with claim 2 or a fragment thereof of an homologous sequence.

27. A plasmid according to claim 25, characterised in that it comprises the said sequence in association with a vector pUC18.

28. A plasmid according to claim 26 or claim 27, characterised in that it has been designated pMTO1 and in that it has been filed at the National Collection of Micro-organism Cultures maintained by the Pasteur Institute on 25th August 1989 under No. I-900.

29. A method for the rapid detection and identification of at least one group and/or species of mycobacteria in a biological specimen, characterised in that it comprises:
(1) a stage in which the biological specimen is brought into contact with at least one diagnostic reagent according to any one of claims 20 to 24;
(2) a stage in which, by any suitable means, the product or products resulting from the interaction of the nucleotide sequence of mycobacteria which may be present with the diagnostic reagent is detected.

30. A process according to claim 29, characterised in that the diaqnostic reagent(s) of stage (1) is/are a pair/pairs of primers according to any one of claims 10 to 13 and make it possible to obtain products for amplifying the nucleotide sequence to be detected.

31. A process according to claim 30, characterised in that the amplifying products obtained in (1) are detected by electrophoretic separation.

32. A process according to claim 30, characterised in that the amplifying products obtained in (1) are detected by hybridisation for two hours at 65°C between the said amplifying products and a diagnostic reagent according to any one of claims 19, 22, 23 and 24.

33. A process according to claim 29, characterised in that the diagnostic reagent of stage (1) is a detection probe according to any one of claims 19, 22, 23 or 24*.*

34. A process for detecting the presence of Mycobacterium tuberculosis in a biological specimen, according to claim 29, characterised by the following stages:
i) establishing contact between the biological specimen and a pair of nucleic acid fragments referred to as primers , according to any one of claims 10 to 13, the DNA contained in the specimen having, if applicable, been previously rendered accessible to hybridisation and under conditions which permit hybridisation of the Mycobacterium tuberculosis DNA primers , such as 2 minutes at 60°C;
ii) amplification of the Mycobacterium tuberculosis DNA;
iii) demonstration of the amplification of fragments of DNA corresponding to the fragment framed by the primers , particularly by electrophoresis over ice;
iv) possible verification of the sequence of amplified fragment, particularly by hybridisation of a specific probe, by sequencing or by analysing the restriction site.

35. A method for the in vitro diagnosis of an infection by mycobacteria on a given biological specimen, according to claim 29, characterised in that it comprises the following stages:
a) establishing contact between the nucleic acid of mycobacteria possibly present in the biological specimen tested, if necessary under conditions which allow accessibility in the form of a simple DNA strand with at least one pair of nucleotide primers according to any one of claims 10 to 13;
b) separation of the synthesised DNA strands from their matrix;
c) repetition of the synthesis of an elongation product starting from each DNA strand present at the end of stage b) and capable of hybridising with the primers until there is the desired amplification of the DNA adequate for detection purposes;
d) establishment of contact between the product stage c) and a nucleotide probe under conditions which make it possible to detect the presence of the desired amplified DNA fragment;
e) detection of any hybridisation products which have possibly been formed.

36. A process for the in vitro diagnosis according to claim 34, characterised in that the establishment of contact with the specimen tested is preceded by a stage involving treatment of the specimen in order to extract the nucleic acid from it.

37. A process according to either one of claims 35 or 36, characterised in that prior to the establishment of contact with the primers , the nucleic acid in the specimen is treated with a reverse-transcriptase to obtain synthesis of the cDNA from the RNA possibly present in the specimen tested.

38. A kit, box or co-ordinated set ready for use and for carrying out the process of detecting at least one group and/or one species of mycobacteria according to any one of claims 29 o 37, characterised in that in addition to useful quantities of buffers and reagents suitable for carrying out the said detection, it comprises:
- suitable doses of a pair of primers according to any one of claims 10 to 13, and/or
- suitable doses of at least one probe or fragment of nucleotide probe according to any one of claims 18, 21, 22 or 23.

39. A kit for the in vitro diagnosis of an infection by mycobacteria on a given biological specimen according to claim 38, characterised in that it comprises:
- at least one pair of nucleotide primers according to any one of claims 10 to 13,
- reagents needed for the extraction of nucleic acids from the specimen treated,
- reagents for carrying out polymerisation of the said DNA fragment on a basis of nucleotide primers , particularly polymerisation enzymes, in a sufficient quantity to produce amplification of the DNA fragment which it is desired to amplify;
- at least one nucleotide fragment capable of being used as a probe and capable of hybridising under specific conditions, 16 to 18 hours at 68°C or 2 hours at 65°C with the amplified DNA fragment,
- internal control of the amplification reaction, consisting for example of a DNA fragment possibly carried by a plasmid, the said fragment being capable of being easily detected, for example in that it contains a gene resistant to an antibiotic, said fragment being furthermore provided at both ends with at least one amplification primer preferably chosen from among the primers according to the invention,
- a probe capable of hybridising with the DNA fragment contained in the internal control,
- if applicable, a reverse transcriptase to obtain cDNA from the RNA possibly present in the specimen tested,
- if applicable, means of revealing the hybridisation.

40. A kit for in vitro diagnosis in accordance with claim 39, characterised in that the nucleotide primers are ISTB2 and ISTB7 and in that at least one detection probe is used which is constituted by the IS-2 marked nucleotide fragment, possibly supplemented by the probe constituted by the IS-6 marked nucleotide fragment.

41. Nucleic acid sequence specific to Mycobacterium tuberculosis, displaying the sequence:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGGGAT GCCCTCATTG GCAACGTTTG CGCCCTGCCT TGGGGGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTGTA GCCGCGCGAT GAACCGCCCC GGCATGTCCG GAGACTCCAG
370 380 390 400 410 420
TTCTTCGAAA GGATGGGGTC ATGTCAGGTG GTTCATCCAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGGCGAACGC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
CTGCACACAG CTGACCGAGC TGGGTGTGCC GATCGCCCCA TCGACCTACT ACGACCACAT
850 860 870 880 890 900
GAACCGGGAG CCCAGCCGCC GCGAGCTGCG CGATGGCGAA CTCAAGGAGC ACATCAGCCC
910 920 930 940 950 960
CGTCCACGCC GCCAACTACG GTGTTTACGG TGCCCGCAAA GTGTGGCTAA CCCTGAACCG
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCCCAGGAC CACGATCGCT GATCGGGCCA CAGCCCGTCC
1090 1100 1110 1120 1130 1140
CGCCGATCTC GTCCAGCGCC GCTTCGGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CGGTCGGAGC GGTCGGAAGC TCCTATGACA ATGCACTAGC
1450 1460 1470 1480 1490 1500
CGAGACGATC AACGGCCTAT ACAAGACCGA GCTGATCAAA CCCGGCAAGC CCTGGCGGTC
1510 1520 1530 1540 1550 1560
CATCGAGGAT GTCGAGTTGG CCACCGCGCG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACG
1630 1640 1650 1660 1670 1680
CCAGAGACCA GCCCCCGGCT GAGGTCTCAG ATCAGAGAGT CTCCGGACTC ACCGGGGCGG

TTCA

## Claims (Claims for the following Contracting State(s): ES)

1. A process for obtaining a nucleotide sequence derived from mycobacteria, characterised by the preparation of a nucleotide sequence repeated in the genome of a mycobacterium and specific to the group of tuberculosis bacilli and in that it hybridises very strongly with *M.tuberculosis,* that is to say when it is marked with phosphorous 32 it provides a spot visible to the naked eye after autoradiography for one hour and in that it hybridises very weakly with M.bovis BCG, that is to say when it is marked with phosphorous 32 it supplies a spot visible to the naked eye after autoradiography for 48 hours.

2. A process for obtaining a sequence according to claim 1, characterised by the preparation of a sequence comprising at its end 5' the sequence 5'TGAACCGCCCCGG 3' of formula I while at its end 3' it comprises the sequence 5'CCGGGGCGGTTCA 3' of formula II, the said sequence containing additionally at least one fragment of a sequence of the following formula III:

10 20 30 40 50 60
GTCGACACGC CTTCTGCACG GGAAGTCCTT CTGCGGCCAT CGTTGCTATG GCCGCTTACT
70 80 90 100 110 120
GCCTTCTAGT CCGTGCGGCT CTCGCAACAG CTCACGGGAC CTTTTTGAGG ATCGCCACTT
130 140 150 160 170 180
CAGGTCTTCA ACTCGCGGAT GCCCTCATTG GCAACGTTTG CGCCCTGCCT TGGGGCGGCC
190 200 210 220 230 240
GGCAGCCACC AAGTCGAGCA CTTTGCGGCG GAACTACTCG GGGTAACACT TCGGCACGGA
250 260 270 280 290 300
CACGGCTCGT TCGACGGACG TCGTGACCAG AAGTCGAGCA AACCGACTCC ACTCTAGCTA
310 320 330 340 350 360
GTGATACAAG CTTTTTTCTA GCCGCGCGAT GAACCGCCCC GGCATGTCCG GAGACTCCAG
370 380 390 400 410 420
TTCTTGGAAA GGATGGGGTC ATGTCAGGTG GTTCATCGAG GAGGTACCCG CCGGAGCTGC
430 440 450 460 470 480
GTGAGCGGGC GGTGCGGATG GTCGCAGAGA TCCGCGGTCA GCACGATTCG GAGTGGGCAG
490 500 510 520 530 540
CGATCAGTGA GGTCGCCCGT CTACTTGGTG TTGGCTGCGC GGAGACGGTG CGTAAGTGGG
550 560 570 580 590 600
TGCGCCAGGC GCAGGTCGAT GCCGGCGCAC GGCCCGGGAC CACGACCGAA GAATCCGCTG
610 620 630 640 650 660
AGCTGAAGCG CTTAGCGGCG GGACAACGCC GAATTGCGAA GGCGAACGCC GATTTTAAAG
670 680 690 700 710 720
ACCGCGTCGG CTTTCTTCGC GGCCGAGCTC GACCGGCCAG CACGCTAATT AACGGTTCAT
730 740 750 760 770 780
CGCCGATCAT CAGGGCCACC GCGAGGGCCC CGATGGTTTG CGGTGGGGTG TCGAGTCGAT

790 800 810 820 830 840
CTGCACACAG CTGACCCAGC TCGGTGTGCC GATCGCCCCA TCGACCTACT ACGACCACAT
850 860 870 880 890 900
GAACCGGGAG CCCAGCCGCC GCCAGCTGCG CGATGGCGAA CTCAAGGAGC ACATCAGCCC
910 920 930 940 950 960
CGTCCACGCC GCCAACTACG GTGTTTACGG TGCCGGCAAA GTGTGGCTAA CCCTGAACCC
970 980 990 1000 1010 1020
TGAGGGCATC GAGGTGGCCA GATGCACCGT CGAACGGCTG ATGACCAAAC TCGGCCTGTC
1030 1040 1050 1060 1070 1080
CGGGACCACC CGCGGCAAAG CCCCCAGGAC CACGATCGCT GATCCGGCCA CAGCCCGTCC
1090 1100 1110 1120 1130 1140
CGCCGATCTC GTCCAGCGCC GCTTCCGACC ACCAGCACCT AACCGGCTGT GGGTAGCAGA
1150 1160 1170 1180 1190 1200
CCTCACCTAT GTGTCCACCT GGGCAGGGTT CGCCTACGTG GCCTTTGTCA CCGACGCCTA
1210 1220 1230 1240 1250 1260
CGCTCGCAGG ATCCTGGGCT GGCGGGTCGC TTCCACGATG GCCACCTCCA TGGTCCTCGA
1270 1280 1290 1300 1310 1320
CGCGATCGAG CAAGCCATCT GGACCCGCCA ACAAGAAGGC GTACTCGACC TGAAAGACGT
1330 1340 1350 1360 1370 1380
TATCCACCAT ACGGATAGGG GATCTCAGTA CACATCGATC CGGTTCAGCG AGCGGCTCGC
1390 1400 1410 1420 1430 1440
CGAGGCAGGC ATCCAACCGT CCGTCGGAGC GGTCGGAAGC TCCTATGACA ATGCACTAGC
1450 1460 1470 1480 1490 1500
CGAGACGATC AACGGCCTAT ACAAGACCGA GCTCATCAAA CCCGGCAAGC CCTGGCGGTC
1510 1520 1530 1540 1550 1560
CATCGAGGAT GTCCAGTTCG CCACCGCCGG CTGGGTCGAC TGGTTCAACC ATCGCCGCCT
1570 1580 1590 1600 1610 1620
CTACCAGTAC TGCGGCGACG TCCCGCCGGT CGAACTCGAG GCTGCCTACT ACGCTCAACC
1630 1640 1650 1660 1670 1680
GCAGAGACCA GCCGCCCGCT GAGGTCTCAG ATCAGAGAGT CTCCGGACTC ACCGGGGCGG

TTCA

3. A process for obtaining a nucleotide sequence according to claim 2, characterised by the preparation of a sequence comprising the fragment 343-1152 of the sequence of formula III in accordance with claim 2 and any sequence displaying at least 80% homology therewith.

4. A process for obtaining a nucleotide sequence according to claim 2, characterised by the preparation of a sequence comprising the fragment 327-1684 of the sequence of formula III in accordance with claim 2 and any sequence displaying at least 80% homology therewith.

5. A process for obtaining an oligonucleotide, characterised by the preparation of a fragment of a nucleotide sequence according to any one of claims 1 to 4.

6. A process for obtaining an oligonucleotide according to claim 5, characterised by the preparation of an oligonucleotide to the following formula IV:

ATGTCAGGTGGTTCATCGAG (IV)

7. A process for obtaining an oligonucleotide according to claim 5, characterised by preparation of the oligonucleotide to the following formula V:

ACAGGCCGAGTTTGGTCATC (V)

8. A process for obtaining nucleotide fragments according to claim 5, characterised by preparation of a fragment displaying one of the following sequences:

ISTB-3 : 5'TTCGCAATTCTGGCGATTGTCC 3' (VI)
ISTB-4 : 5'TCGACCTACTACGACCACAT 3' (VII)
ISTB-5 : 5'GTCGAGTCGATCTGCACACA 3' (VIII)
ISTB-6 : 5'GTTCAGGGTTAGCCACACTT 3' (IX)
ISTB-7 : 5'CAGCACGCTAATTAACCCGG 3' (X)
ISTB-8 : 5'TGGCCAGATGCACCGTCGAA 3' (XI)
ISTB-9 : 5'AGTACACATCGATCCGGTTC 3' (XII)
ISTB-10: 5'TACTACGCTCAACGCCAGAG 3' (XIII)
IS-2 : 5'CATCAGCCGCGTCCACGCCGCCAACTACGG 3' (XIV)
IS-4 : 5'TGTGCCGATCGCCCCATCGACCTACTACGA 3' (XV)
IS-6 : 5'GTCGAGTCGATCTGCACACAGCTGACCGAG 3' (XVI)

9. A process for obtaining nucleotide fragments, characterised by the preparation of a complementary fragment of the fragments according to claim 8 or fragments modified in comparison with the preceding fragments by removal or addition of nucleotide(s) in a proportion of approximately 10 to 15% in relation to the length of the above fragments and/or by modification of the nature of the nucleotides and hence the modified nucleotide fragments retain a capacity for hybridisation with the DNA sequence of mycobacteria similar to that displayed by the corresponding non-modified fragments.

10. A process for obtaining primers for amplifying DNA fragments of mycobacteria, particularly for amplifying fragments of a sequence of Mycobacterium tuberculosis, characterised by the preparation of a sequence or a fragment of a sequence as defined in any one of claims 6 to 9, chosen from among the following or from their complementary nucleotide fragments:

ISTB1 : 5'ATGTCAGGTGGTTCATCGAG 3'
ISTB2 : 5'ACAGGCCGAGTTTGGTCATC 3'
ISTB3 : 5'TTCGCAATTCTGGCGTTGTCC 3'
ISTB4 : 5'TCGACCTACTACGACCACAT 3'
ISTB5 : 5'GTCGAGTCGATCTGCACACA 3'
ISTB6 : 5'GTTCAGGGTTAGCCACACTT 3'
ISTB7 : 5'CAGCACGCTAATTAACCCGG 3'
ISTB8 : 5'TGGCCAGATGCACCGTCGAA 3'
ISTB9 : 5'AGTACACATCGATCCGGTTC 3'
ISTB10: 5'TACTACGCTCAACGCCAGAG 3'

11. A process for obtaining primers for amplifying DNA fragments of mycobacteria according to claim 10, characterised by the preparation of the following pairs of primers :

ISTB2 and ISTB4:
5'ACAGGCCGAGTTTGGTCATC 3' and
5'TCGACCTACTACGACCACAT 3'

ISTB2 and ISTB5:
5'ACAGGCCGAGTTTGGTCATC 3' and
5'GTCGAGTCGATCTGCACACA 3'
ISTB5 and ISTB6:
5'GTCGAGTCGATCTGCACACA 3' and
5'GTTCAGGGTTAGCCACACTT 3'
ISTB6 and ISTB7:
5'GTTCAGGGTTAGCCACACTT 3' and
5'CAGCACGCTAATTAACCCGG 3'.

12. A process for obtaining primers for amplifying DNA fragments according to claim 10 or claim 11, characterised by the preparation of the following pair of primers :

ISTB2 and ISTB7:
5'ACAGGCCGAGTTTGGTCATC 3' and
5'CAGCACGCTAATTAACCCGG 3'.

13. A process for obtaining primers according to any one of claims 10 to 12, characterised in that the said primers are marked by a chemical, physical or enzymatic marker and/or in that they are fixed on a solid carrier, especially a particulate or membrane carrier, in particular magnetic balls.

14. A process for obtaining products of translation and/or fragments thereof, characterised in that the said products are prepared on the basis of a sequence or a fragment of a nucleotide sequence according to any one of claims 1 to 4.

15. A process for obtaining a nucleic probe capable of detecting by hybridisation the presence of DNA specific to Mycobacterium tuberculosis in a biological specimen, characterised by the preparation of a probe having a sequence with a length of at least 20 bases, capable of hybridising, after being brought into contact for 16 to 18 hours at 68°C or for 2 hours at 65°, with a part of the sequence constituted by the fragment 327-1684 in accordance with claim 4, and situated between the two nucleic primers.

16. A process for obtaining a probe according to claim 15, characterised by the preparation of one of the following nucleotide fragments:

IS-2 : 5'CATCAGCCGCGTCCACGCCGCCAACTACGG 3'
IS-4 : 5'TGTGCCGATCGCCCCATCGACCTACTACGA 3'
IS-6 : 5'GTCGAGTCGATCTGCACACAGCTGACCGAG 3'.

17. A process for obtaining a probes for the detection of amplified DNA fragments of mycobacteria according to claim 15, characterised by the preparation of nucleotide fragments specific to a mycobacteria sequence according to any one of claims 5 to 8, capable of hybridising with the said amplified DNA fragment after contact for 16 to 18 hours at 68°C or for 2 hours at 65°C, the said fragments being either marked at their end 5' and/or 3' by a substance which is possible to detect, for example by a radioactive isotope, an enzyme, a suitable chemical or chemico-luminescent marker, a fluorochrome, a hapten or an antibody, analogues of bases or even a physical marker, or fixed to a solid carrier, especially a particulate or membrane type of carrier, for example magnetic balls.

18. A process for detecting a repeated sequence specific to the group of the tuberculosis bacillus according to any one of claims 1 to 4, characterised in that it comprises:
(1) a stage in which a recombining cosmide bank is prepared comprising fragments of DNA of a suitable mycobacterium in excess of 30 kb, and
(2) a stage in which the cosmide clones containing at least one repeated sequence is/are detected by hybridisation for 16 to 18 hours at 68°C with a total genome DNA of a suitable mycobacterium marked in an appropriate manner.

19. A process for obtaining a diagnostic reagent for detecting at least one group of mycobacteria, characterised by the preparation of the said reagent on a basis of a nucleotide sequence or a fragment thereof in accordance with any one of claims 1 to 8 for detecting mycobacteria of the tuberculosis bacillus group, possibly associated with at least one other nucleotide sequence suitable for detecting another group of mycobacteria and/or with at least one suitable marker.

20. A process for obtaining a reagent according to claim 19, characterised by the preparation of pairs of primers for the synthesis of a fragment of DNA or RNA of the tuberculosis bacillus group, each primer comprising a sequence or a fragment of a nucleotide sequence according to any one of claims 1 to 8.

21. A process for obtaining a reagent according to claim 20, characterised by the preparation of a pair of primers comprising oligonucleotides paired in accordance with claims 10 to 13.

22. A process for obtaining a reagent according to claim 19, characterised by the preparation of a suitable probe for detecting a fragment of DNA or RNA of the tuberculosis bacillus group.

23. A process for obtaining a reagent according to claim 22, characterised by the preparation of a nucleotide sequence of formula III according to any one of claims 2 to 4.

24. A process for obtaining a reagent according to claim 19 or claim 22, characterised in that the marker is chosen from the group comprising in particular the radioactive isotopes, the suitable enzymes, the fluorochromes, the suitable chemical markers, the haptens and the antibodies or the suitable base analogues.

25. A process for obtaining a family of recombining plasmids, characterised in that a nucleotide sequence according to any one of claims 1 to 4 is introduced into a suitable plasmid.

26. A process for obtaining a plasmid according to claim 25, characterised in that the nucleotide sequence of formula III according to claim 2 or a fragment thereof or an homologous sequence is introduced into a suitable plasmid.

27. A process for obtaining a plasmid according to claim 25, characterised in that the said sequences associates with a vector pUC18.

28. A process for obtaining a plasmid according to claim 26 or claim 27, characterised in that the said plasmid, designated pMTO1,has been filed at the National Collection of Micro-organism Cultures maintained by the Pasteur Institute on 25th August 1989 under No. I-900.

29. A method for the rapid detection and identification of at least one group and/or species of mycobacteria in a biological specimen, characterised in that it comprises:
(1) a stage in which the biological specimen is brought into contact with at least one diagnostic reagent according to any one of claims 20 to 24;
(2) a stage in which, by any suitable means, the product or products resulting from the interaction of the nucleotide sequence of mycobacteria which may be present with the diagnostic reagent is detected.

30. A process according to claim 29, characterised in that the diagnostic reagent(s) of stage (1) is/are a pair/pairs of primers according to any one of claims 10 to 13 and make it possible to obtain products for amplifying the nucleotide sequence to be detected.

31. A process according to claim 30, characterised in that the amplifying products obtained in (1) are detected by electrophoretic separation.

32. A process according to claim 30, characterised in that the amplifying products obtained in (1) are detected by hybridisation for two hours at 65°C between the said amplifying products and a diagnostic reagent according to any one of claims 19, 22, 23 and 24.

33. A process according to claim 29, characterised in that the diagnostic reagent of stage (1) is a detection probe according to any one of claims 19, 22, 23 or 24.

34. A process for detecting the presence of Mycobacterium tuberculosis in a biological specimen, according to claim 29, characterised by the following stages:
i) establishing contact between the biological specimen and a pair of nucleic acid fragments referred to as primers , according to any one of claims 10 to 13, the DNA contained in the specimen having, if applicable, been previously rendered accessible to hybridisation and under conditions which permit hybridisation of the Mycobacterium tuberculosis DNA primers , such as 2 minutes at 60°C;
ii) amplification of the Mycobacterium tuberculosis DNA;
iii) demonstration of the amplification of fragments of DNA corresponding to the fragment framed by the primers , particularly by electrophoresis over ice;
iv) possible verification of the sequence of amplified fragment, particularly by hybridisation of a specific probe, by sequencing or by analysing the restriction site.

35. A method for the in vitro diagnosis of an infection by mycobacteria on a given biological specimen, according to claim 29, characterised in that it comprises the following stages:
a) establishing contact between the nucleic acid of mycobacteria possibly present in the biological specimen tested, if necessary under conditions which allow accessibility in the form of a simple DNA strand with at least one pair of nucleotide primers according to any one of claims 10 to 13;
b) separation of the synthesised DNA strands from their matrix;
c) repetition of the synthesis of an elongation product starting from each DNA strand present at the end of stage b) and capable of hybridising with the primers until there is the desired amplification of the DNA adequate for detection purposes;
d) establishment of contact between the product stage c) and a nucleotide probe under conditions which make it possible to detect the presence of the desired amplified DNA fragment;
e) detection of any hybridisation products which have possibly been formed.

36. A process for the in vitro diagnosis according to claim 34, characterised in that the establishment of contact with the specimen tested is preceded by a stage involving treatment of the specimen in order to extract the nucleic acid from it.

37. A process according to either one of claims 35 or 36, characterised in that prior to the establishment of contact with the primers , the nucleic acid in the specimen is treated with a reverse-transcriptase to obtain synthesis of the cDNA from the RNA possibly present in the specimen tested.

38. A kit, box or co-ordinated set ready for use and for carrying out the process of detecting at least one group and/or one species of mycobacteria according to any one of claims 29 o 37, characterised in that in addition to useful quantities of buffers and reagents suitable for carrying out the said detection, it comprises:
- suitable doses of a pair of primers according to any one of claims 10 to 13, and/or
- suitable doses of at least one probe or fragment of nucleotide probe according to any one of claims 18, 21, 22 or 23.

39. A kit for the in vitro diagnosis of an infection by mycobacteria on a given biological specimen according to claim 38, characterised in that it comprises:
- at least one pair of nucleotide primers according to any one of claims 10 to 13,
- reagents needed for the extraction of nucleic acids from the specimen treated,
- reagents for carrying out polymerisation of the said DNA fragment on a basis of nucleotide primers , particularly polymerisation enzymes, in a sufficient quantity to produce amplification of the DNA fragment which it is desired to amplify;
- at least one nucleotide fragment capable of being used as a probe and capable of hybridising under specific conditions, 16 to 18 hours at 68°C or 2 hours at 65°C with the amplified DNA fragment,
- internal control of the amplification reaction, consisting for example of a DNA fragment possibly carried by a plasmid, the said fragment being capable of being easily detected, for example in that it contains a gene resistant to an antibiotic, said fragment being furthermore provided at both ends with at least one amplification primer preferably chosen from among the primers according to the invention,
- a probe capable of hybridising with the DNA fragment contained in the internal control,
- if applicable, a reverse transcriptase to obtain cDNA from the RNA possibly present in the specimen tested,
- if applicable, means of revealing the hybridisation.

40. A kit for in vitro diagnosis in accordance with claim 39, characterised in that the nucleotide primers are ISTB2 and ISTB7 and in that at least one detection probe is used which is constituted by the IS-2 marked nucleotide fragment, possibly supplemented by the probe constituted by the IS-6 marked nucleotide fragment.

41. A process for obtaining a nucleic acid sequence specific to Mycobacterium tuberculosis, characterised by the preparation of a sequence of the following formula:
